(19) Europäisches Patentamt
European Patent Office
Office européen des brevets

(11) EP 1 035 209 A1

(12) EUROPEAN PATENT APPLICATION

(43) Date of publication:
**13.09.2000 Bulletin 2000/37**

(21) Application number: **99104519.6**

(22) Date of filing: **06.03.1999**

(51) Int Cl.[7]: **C12N 15/86**, C12N 7/01, C12N 5/10, A61K 39/00, A61K 39/145, A61K 48/00

(84) Designated Contracting States:
**AT BE CH CY DE DK ES FI FR GB GR IE IT LI LU MC NL PT SE**
Designated Extension States:
**AL LT LV MK RO SI**

(71) Applicant: **ARTEMIS Pharmaceuticals GmbH**
**51063 Köln (DE)**

(72) Inventors:
- **Hobom, Gerd**
  **35392 Giessen (DE)**
- **Flick, Ramon**
  **13793 Västerhaninge (SE)**
- **Menke, Anette**
  **35037 Marburg (DE)**
- **Azzey, Mayra**
  **35394 Giessen (DE)**

(74) Representative:
**Helbing, Jörg, Dr. Dipl.-Chem. et al**
**Patentanwälte**
**von Kreisler-Selting-Werner,**
**Postfach 10 22 41**
**50462 Köln (DE)**

(54) **Stable recombinant influenza viruses free of helper viruses**

(57) The invention relates to a recombinant influenza virus for high-yield expression of incorporated foreign gene(s), which is genetically stable in the absence of any helper virus; wherein

(a) at least one of the regular viral RNA segments has been exchanged for a vRNA encoding a foreign gene; and/or
(b) at least one of the regular viral RNA segments is an ambisense RNA molecule, containing one of the standard viral genes in sense orientation and a foreign, recombinant gene in antisense orientation, or *vice versa,* in overall convergent arrangement.

The present invention further provides a method for the production of said recombinant influenza virus, a method for constructing specific ribozyme-sensitive influenza carrier strains; pharmaceutical compositions comprising said recombinant influenza virus; and the use of said recombinant influenza virus for preparing medicaments for vaccination purposes.

EP 1 035 209 A1

SEQUENCE LISTING

<110> ARTEMIS PHARMACEUTICALS GmbH

<120> Stable Recombinant Influenza Viruses Free of Helper Viruses

<130> 990383ep

<140>
<141>

<160> 9

<170> PatentIn Ver. 2.1

<210> 1
<211> 4930
<212> DNA
<213> Influenza virus

<400> 1
cgtacgaagc ttctagaggg attggctgag acgaaaaaca tatgctagag ggattggctg 60
agacgaaaaa catatgctag agcggccgcc accgcggtgg agctccagct tttgttccct 120
ttagtgaggg ttaattgcgc gcaggcctag ctaggtaaag aaaaataccc ttgtttctac 180
taataacccg gcggcccaaa atgccgactc ggagcgaaag atatacctcc cccggggccg 240
ggaggtcgcg tcaccgacca cgccgccggc ccaggcgacg cgcgacacgg acacctgtcc 300
ccaaaaacgc caccatcgca gccacacacg gagcgcccgg ggccctctgg tcaaccccag 360
gacacacgcg ggagcagcgc cgggccgggg acgccctccc ggccgcccgt gccacacgca 420
gggggccggc ccgtgtctcc agagcgggag ccggaagcat ttcggccgg ccccctcctac 480
gaccgggaca cacgagggac cgaaggccgg ccaggcgcga cctctcgggc cgcacgcgcg 540
ctcagggagc gctctccgac tccgcacggg gactcgccag aaaggatcgt gacctgcatt 600
aatgaatcag gggataacgc aggaaagaac atgtgagcaa aaggccagca aaaggccagg 660
aaccgtaaaa aggccgcgtt gctggcgttt ttccataggc tccgcccccc tgacgagcat 720
cacaaaaatc gacgctcaag tcagaggtgg cgaaacccga caggactata aagataccag 780
gcgtttcccc ctggaagctc cctcgtgcgc tctcctgttc cgaccctgcc gcttaccgga 840
tacctgtccg cctttctccc ttcgggaagc gtggcgcttt ctcatagctc acgctgtagg 900
tatctcagtt cggtgtaggt cgttcgctcc aagctgggct gtgtgcacga accccccgtt 960
cagcccgacc gctgcgcctt atccggtaac tatcgtcttg agtccaaccc ggtaagacac 1020
gacttatcgc cactggcagc agccactggt aacaggatta gcagagcgag gtatgtaggc 1080
ggtgctacag agttcttgaa gtggtggcct aactacggct acactagaag gacagtattt 1140
ggtatctgcg ctctgctgaa gccagttacc ttcggaaaaa gagttggtag ctcttgatcc 1200
ggcaaacaaa ccaccgctgg tagcggtggt ttttttgttt gcaagcagca gattacgcgc 1260
agaaaaaaag gatctcaaga agatcctttg atcttttcta cggggtctga cgctcagtgg 1320
aacgaaaact cacgttaagg gattttggtc atgagattat caaaaaggat cttcacctag 1380
atccttttaa attaaaaatg aagttttaaa tcaatctaaa gtatatatga gtaaacttgg 1440
tctgacagtt accaatgctt aatcagtgag gcacctatct cagcgatctg tctatttcgt 1500
tcatccatag ttgcctgact ccccgtcgtg tagataacta cgatacggga gggcttacca 1560
tctggcccca gtgctgcaat gataccgcga gacccacgct caccggctcc agatttatca 1620
gcaataaacc agccagccgg aagggccgag cgcagaagtg gtcctgcaac tttatccgcc 1680
tccatccagt ctattaattg ttgccgggaa gctagagtaa gtagttcgcc agttaatagt 1740
ttgcgcaacg ttgttgccat tgctacaggc atcgtggtgt cacgctcgtc gtttggtatg 1800
gcttcattca gctccggttc ccaacgatca aggcgagtta catgatcccc catgttgtgc 1860
aaaaaagcgg ttagctcctt cggtcctccg atcgttgtca gaagtaagtt ggccgcagtg 1920
ttatcactca tggttatggc agcactgcat aattctctta ctgtcatgcc atccgtaaga 1980

```
tgcttttctg tgactggtga gtactcaacc aagtcattct gagaatagtg tatgcggcga 2040
ccgagttgct cttgcccggc gtcaacacgg gataataccg cgccacatag cagaacttta 2100
aaagtgctca tcattggaaa acgttcttcg gggcgaaaac tctcaaggat cttaccgctg 2160
ttgagatcca gttcgatgta acccactcgt gcacccaact gatcttcagc atcttttact 2220
ttcaccagcg tttctgggtg agcaaaaaca ggaaggcaaa atgccgcaaa aaagggaata 2280
agggcgacac ggaaatgttg aatactcata ctcttccttt ttcaatatta ttgaagcatt 2340
tatcagggtt attgtctcat gagcggatac atatttgaat gtatttagaa aaataaacaa 2400
aagagtttgt agaaacgcaa aaaggccatc cgtcaggatg gccttctgct taatttgatg 2460
cctggcagtt tatggcgggc gtcctgcccg ccaccctccg ggccgttgct tcgcaacgtt 2520
caaatccgct cccggcggat ttgtcctact caggagagcg ttcaccgaca aacaacagat 2580
aaaacgaaag gcccagtctt tcgactgagc ctttcgtttt atttgatgcc tggcagttcc 2640
ctactctcgc atggggagac cccacactac catcggcgct acggcgtttc acttctgagt 2700
tcggcatggg gtcaggtggg accaccgcgc tactgccgcc aggcaaattc tgttttatca 2760
gaccgcttct gcgttctgat ttaatctgta tcaggctgaa aatcttctct catccgccaa 2820
aacagaagct agcggccgat ccccaaaaaa aaaaaaaaaa aaaaaaaaaa gagtccagag 2880
tggccccgcc gttccgcgcc gggggggggg gggggggggg acactttcgg acatctggtc 2940
gacctccagc atcgggggaa aaaaaaaaaa caaagtttcg cccggagtac tggtcgacct 3000
ccgaagttgg gggggagtag aaacagggta gataatcact cactgacgta cgttgagcaa 3060
ctgactgaaa tgccttgagc aactgactga aatgcctgac gtctttagca aaagcagggt 3120
agataatcac tcactgagtg acatccacat cgtaccagga ttggctgaga cgaaaaacat 3180
attgtaccag ggattggctg agacgaaaaa catattgtag gtaccaaaat gaacactcaa 3240
atcctggttt tcgcccttgc ggcagtcatc cccacaaatg cagacaaaat ttgtcttgga 3300
catcatgctg tatcaaatgg caccaaagta aacacactca ctgagagagg agtagaagtt 3360
gtcaatgcaa cggaaacagt ggagcggaca aacatcccca aaatttgctc aaaagggaaa 3420
agaaccactg atcttggcca atgcggactg ttagggacca ttaccggacc acctcaatgc 3480
gaccaatttc tagaattttc agctgatcta ataatcgaga gacgagaagg aaatgatgtt 3540
tgttacccgg ggaagtttgt taatgaagag gcattgcgac aaatcctcag aggatcaggt 3600
gggattgaca aagaaacaat gggattcaca tatagtggaa taaggaccaa cggaacaact 3660
agtgcatgta gaagatcagg gtcttcattc tatgcagaaa tggagtggct cctgtcaaat 3720
acagacaatg cttctttccc acaaatgaca aaatcataca aaaacacagg gagagaatca 3780
gctctgatag tctggggaat ccaccattca ggatcaacca ccgaacagac caaactatat 3840
gggagtggaa ataaactgat aacagtcggg agttccaaat atcatcaatc ttttgtgccg 3900
agtccaggaa cacgaccgca gataaatggc cggtccggac ggattgattt tcattggttg 3960
atcttggatc ccaatgatac agttactttt agtttcaatg gggctttcat agctccaaat 4020
cgtgccagct tcttgagggg aaagtccatg gggatccaga gcgatgtgca ggttgatgct 4080
aattgcgaag ggaatgcta ccacagtgga gggactataa caagcagatt gccttttcaa 4140
aacataaata gcagagcagt tggcaaatgc ccaagatatg taaaacagga aagtttatta 4200
ttggcaactg ggatgaagaa cgttcccgaa ccttccaaaa aaaggaaaaa aagaggcctg 4260
tttggtgcta tagcagggtt tattgaaaat ggttgggaag gtctggtcga cgggtggtac 4320
ggtttcaggc atcagaatgc acaaggagaa ggaactgcag cagactacaa aagcacccaa 4380
tcggcaattg atcagataac cggaaagtta aatagactca ttaagaaaac caaccagcaa 4440
tttgagctaa tagataatga attcactgaa gtggaaaagc agattggcaa tttaattaac 4500
tggaccaaag actccatcac agaagtatgg tcttacaatg ctgaacttct tgtggcaatg 4560
gaaaaccagc acactattga tttggctgat tcagagatga acaagctgta tgagcgagtg 4620
aggaaacaat taagggaaaa tgctgaagag gatggcactg gttgctttga aatttttcat 4680
aaatgtgacg atgattgtat ggctagtata aggaacaata cttatgatca cagcaaatac 4740
agagaagaag cgatgcaaaa tagaatacaa attgacccag tcaaattgag tagtggctac 4800
aaagatgtga tactttggtt tagcttcggg gcatcatgct tttgcttctt tgccattgca 4860
atgggccttg ttttcatatg tgtgaagaac ggaaacatgc ggtgcactat atgcatttaa 4920
agcttgcatg 4930
```

<210> 2
<211> 5811
<212> DNA
<213> Influenza virus

<400> 2

```
aattcctttg cctaatttaa atgaggactt aacctgtgga aatattttga tgtgggaagc 60
tgttactgtt aaaactgagg ttattggggt aactgctatg ttaaacttgc attcagggac 120
acaaaaaact catgaaaatg gtgctggaaa acccattcaa gggtcaaatt ttcatttttt 180
tgctgttggt ggggaacctt tggagctgca gggtgtgtta gcaaactaca ggaccaaata 240
tcctgctcaa actgtaaccc caaaaaatgc tacagttgac agtcagcaga tgaacactga 300
ccacaaggct gttttggata aggataatgc ttatccagtg gagtgctggg ttcctgatcc 360
aagtaaaaat gaaaacacta gatattttgg aacctacaca ggtggggaaa atgtgcctcc 420
tgttttgcac attactaaca cagcaaccac agtgcttctt gatgagcagg gtgttgggcc 480
cttgtgcaaa gctgacagct tgtatgtttc tgctgttgac atttgtgggc tgtttaccaa 540
cacttctgga acacagcagt ggaagggact tcccagatat tttaaaatta cccttagaaa 600
gcggtctgtg aaaaacccct acccaatttc ctttttgtta agtgacctaa ttaacaggag 660
gacacagagg gtggatgggc agcctatgat tggaatgtcc tctcaagtag aggaggttag 720
ggtttatgag gacacagagg agcttcctgg ggatccagac atgataagat acattgatga 780
gtttggacaa accacaacta gaatgcagtg aaaaaaatgc tttatttgtg aaatttgtga 840
tgctattgct ttatttgtaa ccattataag ctgcaataaa caagttaaca acaacaattg 900
cattcatttt atgtttcagg ttcaggggga ggtgtgggag gtttttttaaa gcaagtaaaa 960
cctctacaaa tgtggtatgg ctgattatga tctctagtca aggcactata catcaaatat 1020
tccttattaa cccctttaca aattaaaaag ctaaaggtac acaatttttg agcatagtta 1080
ttaatagcag acactctatg cctgtgtgga gtaagaaaaa acagtatgtt atgattataa 1140
ctgttatgcc tacttataaa ggttacagaa tatttttcca taattttctt gtatagcagt 1200
gcagcttttt cctttgtggt gtaaatagca aagcaagcaa gagttctatt actaaacaca 1260
gcatgactca aaaaacttag caattctgaa ggaaagtcct tggggtcttc tacctttctc 1320
ttcttttttg gaggagtaga atgttgagag tcagcagtag cctcatcatc actagatggc 1380
atttcttctg agcaaaacag gttttcctca ttaaaggcat tccaccactg ctcccattca 1440
tcagttccat aggttggaat ctaaaataca caaacaatta gaatcagtag tttaacacat 1500
tatacactta aaaattttat atttacctta gagctttaaa tctctgtagg tagtttgtcc 1560
aattatgtca caccacagaa gtaaggttcc ttcacaaaga tccgggacca aagcggccat 1620
cgtgcctccc cactcctgca gttcgggggc atggatgcgc ggatagccgc tgctggtttc 1680
ctggatgccg acggatttgc actgccggta gaactccgcg aggtcgtcca gcctcaggca 1740
gcagctgaac caactcgcga ggggatcgag cccggggtgg gcgaagaact ccagcatgag 1800
atccccgcgc tggaggatca tccagccggc gtcccggaaa acgattccga agcccaacct 1860
ttcatagaag gcggcggtgg aatcgaaatc tcgtgatggc aggttgggcg tcgcttggtc 1920
ggtcatttcg atgaattcga gctcggtacc cggggatcct ctagaggcat ttcagtttcg 1980
tcctcacgga ctcatcagag ttgctcaatt cgaaccccag agtcccgctc agaagaactc 2040
gtcaagaagg cgatagaagg cgatgcgctg cgaatcggga gcggcgatac cgtaaagcac 2100
gaggaagcgg tcagcccatt cgccgccaag ctcttcagca atatcacggg tagccaacgc 2160
tatgtcctga tagcggtccg ccacacccag ccggccacag tcgatgaatc cagaaaagcg 2220
gccattttcc accatgatat tcggcaagca ggcatcgcca tgggtcacga cgagatcctc 2280
gccgtcgggc atgcgcgcct tgagcctggc gaacagttcg gctggcgcga gcccctgatg 2340
ctcttcgtcc agatcatcct gatcgacaag accggcttcc atccgagtac gtgctcgctc 2400
gatgcgatgt ttcgcttggt ggtcgaatgg gcaggtagcc ggatcaagcg tatgcagccg 2460
ccgcattgca tcagccatga tggatacttt ctcggcagga gcaaggtgag atgacaggag 2520
atcctgcccc ggcacttcgc ccaatagcag ccagtccctt cccgcttcag tgacaacgtc 2580
gagcacagct gcgcaaggaa cgcccgtcgt ggccagccac gatagccgcg ctgcctcgtc 2640
ctgcagttca ttcagggcac cggacaggtc ggtcttgaca aaaagaaccg ggcgcccctg 2700
cgctgacagc cggaacacgg cggcatcaga gcagccgatt gtctgttgtg cccagtcata 2760
gccgaatagc ctctccaccc aagcggccgg agaacctgcg tgcaatccat cttgttcaat 2820
catgcgaaac gatcctcatc ctgtctcttg atcagatctt gatcccctgc gccatcagat 2880
ccttggcggc aagaaagcca tccagtttac tttgcagggc ttcccaacct taccagaggg 2940
cgccccagct ggcaattccg gttcgcttgc tgtccataaa accgcccagt ctagctatcg 3000
ccatgtaagc ccactgcaag ctacctgctt tctctttgcg cttgcgtttt cccttgtcca 3060
gatagcccag tagctgacat tcatccgggg tcagcaccgt ttctgcggac tggctttcta 3120
cgtgttccgc ttcctttagc agcccttgcg ccctgagtgc ttgcggcagc gtgaagcttt 3180
```

```
ttgcaaaagc ctaggcctcc aaaaaagcct cctcactact tctggaatag ctcagaggcc 3240
gaggcggcct cggcctctgc ataaataaaa aaaattagtc agccatgggg cggagaatgg 3300
gcggaactgg gcggagttag gggcgggatg ggcggagtta ggggcgggac tatggttgct 3360
gactaattga gatgcatgct ttgcatactt ctgcctgctg gggagcctgg ggactttcca 3420
cacctggttg ctgactaatt gagatgcatg ctttgcatac ttctgcctgc tggggagcct 3480
ggggactttc cacaccctaa ctgacacaca ttccacagct gcctcgcgcg tttcggtgat 3540
gacggtgaaa acctctgaca catgcagctc ccggagacgg tcacagcttg tctgtaagcg 3600
gatgccggga gcagacaagc ccgtcagggc gcgtcagcgg gtgttggcgg gtgtcggggc 3660
gcagccatga cccagtcacg tagcgatagc ggagtgtata ctggcttaac tatgcggcat 3720
cagagcagat tgtactgaga gtgcaccata tgcggtgtga aataccgcac agatgcgtaa 3780
ggagaaaata ccgcatcagg cgctcttccg cttcctcgct cactgactcg ctgcgctcgg 3840
tcgttcggct gcggcgagcg gtatcagctc actcaaaggc ggtaatacgg ttatccacag 3900
aatcagggga taacgcagga aagaacatgt gagcaaaagg ccagcaaaag gccaggaacc 3960
gtaaaaaggc cgcgttgctg gcgtttttcc ataggctccg cccccctgac gagcatcaca 4020
aaaatcgacg ctcaagtcag aggtggcgaa acccgacagg actataaaga taccaggcgt 4080
ttccccctgg aagctccctc gtgcgctctc ctgttccgac cctgccgctt accggatacc 4140
tgtccgcctt tctcccttcg ggaagcgtgg cgctttctca tagctcacgc tgtaggtatc 4200
tcagttcggt gtaggtcgtt cgctccaagc tgggctgtgt gcacgaaccc cccgttcagc 4260
ccgaccgctg cgccttatcc ggtaactatc gtcttgagtc caacccggta agacacgact 4320
tatcgccact ggcagcagcc actggtaaca ggattagcag agcgaggtat gtaggcggtg 4380
ctacagagtt cttgaagtgg tggcctaact acggctacac tagaaggaca gtatttggta 4440
tctgcgctct gctgaagcca gttaccttcg gaaaaagagt tggtagctct tgatccggca 4500
aacaaaccac cgctggtagc ggtggttttt ttgtttgcaa gcagcagatt acgcgcagaa 4560
aaaaaggatc tcaagaagat cctttgatct tttctacggg gtctgacgct cagtggaacg 4620
aaaactcacg ttaagggatt ttggtcatga gattatcaaa aaggatcttc acctagatcc 4680
ttttaaatta aaaatgaagt tttaaatcaa tctaaagtat atatgagtaa acttggtctg 4740
acagttacca atgcttaatc agtgaggcac ctatctcagc gatctgtcta tttcgttcat 4800
ccatagttgc ctgactcccc gtcgtgtaga taactacgat acgggagggc ttaccatctg 4860
gccccagtgc tgcaatgata ccgcgagacc cacgctcacc ggctccagat ttatcagcaa 4920
taaaccagcc agccggaagg gccgagcgca gaagtggtcc tgcaacttta tccgcctcca 4980
tccagtctat taattgttgc cgggaagcta gagtaagtag ttcgccagtt aatagtttgc 5040
gcaacgttgt tgccattgct gcaggcatcg tggtgtcacg ctcgtcgttt ggtatggctt 5100
cattcagctc cggttcccaa cgatcaaggc gagttacatg atcccccatg ttgtgcaaaa 5160
aagcggttag ctccttcggt cctccgatcg ttgtcagaag taagttggcc gcagtgttat 5220
cactcatggt tatggcagca ctgcataatt ctcttactgt catgccatcc gtaagatgct 5280
tttctgtgac tggtgagtac tcaaccaagt cattctgaga atagtgtatg cggcgaccga 5340
gttgctcttg cccggcgtca acacgggata ataccgcgcc acatagcaga actttaaaag 5400
tgctcatcat tggaaaacgt tcttcggggc gaaaactctc aaggatctta ccgctgttga 5460
gatccagttc gatgtaaccc actcgtgcac ccaactgatc ttcagcatct tttactttca 5520
ccagcgtttc tgggtgagca aaaacaggaa ggcaaaatgc cgcaaaaaag ggaataaggg 5580
cgacacggaa atgttgaata ctcatactct tcctttttca atattattga agcatttatc 5640
agggttattg tctcatgagc ggatacatat ttgaatgtat ttagaaaaat aaacaaatag 5700
gggttccgcg cacatttccc cgaaaagtgc cacctgacgt ctaagaaacc attattatca 5760
tgacattaac ctataaaaat aggcgtatca cgaggccctt tcgtcttcaa g          5811
```

<210> 3
<211> 2005
<212> DNA
<213> Influenza virus

<400> 3

```
agtagaaaca agggtatttt tctttaccta gctaggcctg cgcgcaatta accctcacta 60
aagggaacaa aagctggagc tccaccgcgg tggcggccgc tctagcatat gtttttcgtc 120
tcagccaatc cctctagcat atgtttttcg tctcagccaa tccctctaga agcttcgtac 180
gcatgcaagc tttaaatgca tatagtgcac cgcatgtttc cgttcttcac acatatgaaa 240
```

acaaggccca ttgcaatggc aagaagcaaa aagcatgatg ccccgaagct aaaccaaagt 300
atcacatctt tgtagccact actcaatttg actgggtcaa tttgtattct attttgcatc 360
gcttcttctc tgtatttgct gtgatcataa gtattgttcc ttatactagc catacaatca 420
tcgtcacatt tatgaaaaat ttcaaagcaa ccagtgccat cctcttcagc atttccctt 480
aattgtttcc tcactcgctc atacagcttg ttcatctctg aatcagccaa atcaatagtg 540
tgctggtttt ccattgccac aagaagttca gcattgtaag accatacttc tgtgatggag 600
tctttggtcc agttaattaa attgccaatc tgcttttcca cttcagtgaa ttcattatct 660
attagctcaa attgctggtt ggttttctta atgagtctat ttaactttcc ggttatctga 720
tcaattgccg attgggtgct tttgtagtct gctgcagttc cttctccttg tgcattctga 780
tgcctgaaac cgtaccaccc gtcgaccaga ccttcccaac cattttcaat aaaccctgct 840
atagcaccaa acaggcctct ttttttcctt tttttggaag gttcgggaac gttcttcatc 900
ccagttgcca ataataaact ttcctgtttt acatatcttg ggcatttgcc aactgctctg 960
ctatttatgt tttgaaaagg caatctgctt gttatagtcc ctccactgtg gtagcattcc 1020
ccttcgcaat tagcatcaac ctgcacatcg ctctggatcc ccatggactt tcccctcaag 1080
aagctggcac gatttggagc tatgaaagcc ccattgaaac taaaagtaac tgtatcattg 1140
ggatccaaga tcaaccaatg aaaatcaatc cgtccggacc ggccatttat ctgcggtcgt 1200
gttcctggac tcggcacaaa agattgatga tatttggaac tcccgactgt tatcagttta 1260
tttccactcc catatagttt ggtctgttcg gtggttgatc ctgaatggtg gattccccag 1320
actatcagag ctgattctct ccctgtgttt ttgtatgatt ttgtcatttg tgggaaagaa 1380
gcattgtctg tatttgacag gagccactcc atttctgcat agaatgaaga ccctgatctt 1440
ctacatgcac tagttgttcc gttggtcctt attccactat atgtgaatcc cattgtttct 1500
ttgtcaatcc cacctgatcc tctgaggatt tgtcgcaatg cctcttcatt aacaaacttc 1560
cccgggtaac aaacatcatt tccttctcgt ctctcgatta ttagatcagc tgaaaattct 1620
agaaattggt cgcattgagg tggtccggta atggtcccta acagtccgca ttggccaaga 1680
tcagtggttc ttttcccttt tgagcaaatt ttggggatgt ttgtccgctc cactgtttcc 1740
gttgcattga caacttctac tcctctctca gtgagtgtgt ttactttggt gccatttgat 1800
acagcatgat gtccaagaca aattttgtct gcatttgtgg ggatgactgc cgcaagggcg 1860
aaaaccagga tttgagtgtt cattttggta cctacaatat gtttttcgtc tcagccaatc 1920
cctggtacaa tatgtttttc gtctcagcca atcctggtac gatgtggatg tcactcagtg 1980
agtgattatc taccctgctt ttgct 2005

<210> 4
<211> 1146
<212> DNA
<213> Influenza virus

<400> 4
agtagaaaca agggtatttt tctttaccta gctaggcctg cgcgcaatta accctcacta 60
aagggaacaa aagctggagc tccaccgcgg tggcggccgc tctagcatat gtttttcgtc 120
tcagccaatc cctctagcat atgtttttcg tctcagccaa tccctctaga agcttcgtac 180
gcatgcttaa ataagctgaa acgagaaagt tcttatctct tgctccactt caagcggtag 240
ttgtaaggct tgcataaatg ttatttgttc aaaactattc tctgttatct tcaatctatg 300
tctcacttct tcaattaacc atcttatttc ttcaaatttc tgactcaatt gttctcgcca 360
ttttccgttt ctgctttgga gggagtggag gtcccccatt ctcattactg cttctccaag 420
cgaatctctg tatagtttca gagactcgaa ctgtgttatc attccattca agtcctccga 480
tgaggacccc aattgcattt ttgacatcct catcagtatg tcctggaaga gaaggcaatg 540
gtgaaatttc gccgacaatt gctccctcat cggttaaagc ccttaatagt atgagagttt 600
ccagccgatc gaaaatcaca ctgaagtttg ctttcagtat gatgttcttc cccatgatcg 660
cctggtccat tctgatgcaa agggagcctg ccactttctg tttgggcatg agcatgaacc 720
agtcccttga catctcttca agagtcatgt cagttaggta gcgtgtagca ggtacagagg 780
caatggtcat tttaagtgcc tcatcggatt cgtcctccag aatccgctcc actatctgct 840
ttccaacacg agtagctgtg tcgatgtcca gaccaagagt gctgcctctt cccctcaggg 900
acttctgatc tcggcgaagt cggtcaagga atggggcatc acccatttct tggtctgcaa 960
atcgtttgcg gacatgccaa agaaagcagt ctacctgaaa gcttgacaca gtgttggaat 1020
ccattatggt acctacaata tgtttttcgt ctcagccaat ccctggtaca atatgttttt 1080

cgtctcagcc aatcctggta cgatgtggat gtcactcagt gagtgattat ctaccctgct 1140
tttgct 1146

<210> 5
<211> 5860
<212> DNA
<213> Influenza virus

<400> 5
catcgattgg ctgactgatg agtccgtgag gacgaaacga aaaacatatt gtagagctcg 60
aattcatcga aatgaccgac caagcgacgc ccaacctgcc atcacgagat ttcgattcca 120
ccgccgcctt ctatgaaagg ttgggcttcg gaatcgtttt ccgggacgcc ggctggatga 180
tcctccagcg cggggatctc atgctggagt tcttcgccca ccccgggctc gatcccctcg 240
cgagttggtt cagctgctgc ctgaggctgg acgacctcgc ggagttctac cggcagtgca 300
aatccgtcgg catccaggaa accagcagcg gctatccgcg catccatgcc cccgaactgc 360
aggagtgggg aggcacgatg gccgctttgg tcccggatct ttgtgaagga accttacttc 420
tgtggtgtga cataattgga caaactacct acagagattt aaagctctaa ggtaaatata 480
aaatttttaa gtgtataatg tgttaaacta ctgattctaa ttgtttgtgt attttagatt 540
ccaacctatg gaactgatga atgggagcag tggtggaatg cctttaatga ggaaaacctg 600
ttttgctcag aagaaatgcc atctagtgat gatgaggcta ctgctgactc tcaacattct 660
actcctccaa aaaagaagag aaaggtagaa gaccccaagg actttccttc agaattgcta 720
agtttttga gtcatgctgt gtttagtaat agaactcttg cttgctttgc tatttacacc 780
acaaaggaaa aagctgcact gctatacaag aaaattatgg aaaaatattc tgtaaccttt 840
ataagtaggc ataacagtta taatcataac atactgtttt ttcttactcc acacaggcat 900
agagtgtctg ctattaataa ctatgctcaa aaattgtgta cctttagctt tttaatttgt 960
aaaggggtta ataaggaata tttgatgtat agtgccttga ctagagatca taatcagcca 1020
taccacattt gtagaggttt tacttgcttt aaaaaacctc ccacacctcc ccctgaacct 1080
gaaacataaa atgaatgcaa ttgttgttgt taacttgttt attgcagctt ataatggtta 1140
caaataaagc aatagcatca caaatttcac aaataaagca tttttttcac tgcattctag 1200
ttgtggtttg tccaaactca tcaatgtatc ttatcatgtc tggatcccca ggaagctcct 1260
ctgtgtcctc ataaacccta acctcctcta cttgagagga cattccaatc ataggctgcc 1320
catccaccct ctgtgtcctc ctgttaatta ggtcacttaa caaaaaggaa attgggtagg 1380
ggtttttcac agaccgcttt ctaagggtaa ttttaaaata tctgggaagt cccttccact 1440
gctgtgttcc agaagtgttg gtaaacagcc cacaaatgtc aacagcagaa acatacaagc 1500
tgtcagcttt gcacaagggc ccaacaccct gctcatcaag aagcactgtg gttgctgtgt 1560
tagtaatgtg caaaacagga ggcacatttt ccccacctgt gtaggttcca aaatatctag 1620
tgttttcatt tttacttgga tcaggaaccc agcactccac tggataagca ttatccttat 1680
ccaaaacagc cttgtggtca gtgttcatct gctgactgtc aactgtagca ttttttgggg 1740
ttacagtttg agcaggatat ttggtcctgt agtttgctaa cacaccctgc agctccaaag 1800
gttccccacc aacagcaaaa aaatgaaaat ttgacccttg aatgggtttt ccagcaccat 1860
tttcatgagt tttttgtgtc cctgaatgca agtttaacat agcagttacc ccaataacct 1920
cagttttaac agtaacagct tcccacatca aaatatttcc acaggttaag tcctcattta 1980
aattaggcaa aggaattctt gaagacgaaa gggcctcgtg atacgcctat ttttataggt 2040
taatgtcatg ataataatgg tttcttagac gtcaggtggc acttttcggg gaaatgtgcg 2100
cggaacccct atttgtttat ttttctaaat acattcaaat atgtatccgc tcatgagaca 2160
ataaccctga taaatgcttc aataatattg aaaaaggaag agtatgagta ttcaacattt 2220
ccgtgtcgcc cttattccct tttttgcggc attttgcctt cctgtttttg ctcacccaga 2280
aacgctggtg aaagtaaaag atgctgaaga tcagttgggt gcacgagtgg gttacatcga 2340
actggatctc aacagcggta agatccttga gagttttcgc cccgaagaac gttttccaat 2400
gatgagcact tttaaagttc tgctatgtgg cgcggtatta tcccgtgttg acgccgggca 2460
agagcaactc ggtcgccgca tacactattc tcagaatgac ttggttgagt actcaccagt 2520
cacagaaaag catcttacgg atggcatgac agtaagagaa ttatgcagtg ctgccataac 2580
catgagtgat aacactgcgg ccaacttact tctgacaacg atcggaggac cgaaggagct 2640
aaccgctttt ttgcacaaca tgggggatca tgtaactcgc cttgatcgtt gggaaccgga 2700
gctgaatgaa gccataccaa acgacgagcg tgacaccacg atgcctgcag caatggcaac 2760

```
aacgttgcgc aaactattaa ctggcgaact acttactcta gcttcccggc aacaattaat 2820
agactggatg gaggcggata aagttgcagg accacttctg cgctcggccc ttccggctgg 2880
ctggtttatt gctgataaat ctggagccgg tgagcgtggg tctcgcggta tcattgcagc 2940
actggggcca gatggtaagc cctcccgtat cgtagttatc tacacgacgg ggagtcaggc 3000
aactatggat gaacgaaata gacagatcgc tgagataggt gcctcactga ttaagcattg 3060
gtaactgtca gaccaagttt actcatatat actttagatt gatttaaaac ttcattttta 3120
atttaaaagg atctaggtga agatcctttt tgataatctc atgaccaaaa tcccttaacg 3180
tgagttttcg ttccactgag cgtcagaccc cgtagaaaag atcaaaggat cttcttgaga 3240
tccttttttt ctgcgcgtaa tctgctgctt gcaaacaaaa aaaccaccgc taccagcggt 3300
ggtttgtttg ccggatcaag agctaccaac tctttttccg aaggtaactg gcttcagcag 3360
agcgcagata ccaaatactg tccttctagt gtagccgtag ttaggccacc acttcaagaa 3420
ctctgtagca ccgcctacat acctcgctct gctaatcctg ttaccagtgg ctgctgccag 3480
tggcgataag tcgtgtctta ccgggttgga ctcaagacga tagttaccgg ataaggcgca 3540
gcggtcgggc tgaacggggg gttcgtgcac acagcccagc ttggagcgaa cgacctacac 3600
cgaactgaga tacctacagc gtgagctatg agaaagcgcc acgcttcccg aagggagaaa 3660
ggcggacagg tatccggtaa gcggcagggt cggaacagga gagcgcacga gggagcttcc 3720
agggggaaac gcctggtatc tttatagtcc tgtcgggttt cgccacctct gacttgagcg 3780
tcgatttttg tgatgctcgt caggggggcg gagcctatgg aaaaacgcca gcaacgcggc 3840
ctttttacgg ttcctggcct tttgctggcc ttttgctcac atgttctttc ctgcgttatc 3900
ccctgattct gtggataacc gtattaccgc ctttgagtga gctgataccg ctcgccgcag 3960
ccgaacgacc gagcgcagcg agtcagtgag cgaggaagcg gaagagcgcc tgatgcggta 4020
ttttctcctt acgcatctgt gcggtatttc acaccgcata tggtgcactc tcagtacaat 4080
ctgctctgat gccgcatagt taagccagta tacactccgc tatcgctacg tgactgggtc 4140
atggctgcgc cccgacaccc gccaacaccc gctgacgcgc cctgacgggc ttgtctgctc 4200
ccggcatccg cttacagaca agctgtgacc gtctccggga gctgcatgtg tcagaggttt 4260
tcaccgtcat caccgaaacg cgcgaggcag ctgtggaatg tgtgtcagtt agggtgtgga 4320
aagtccccag gctccccagc aggcagaagt atgcaaagca tgcatctcaa ttagtcagca 4380
accaggtgtg gaaagtcccc aggctcccca gcaggcagaa gtatgcaaag catgcatctc 4440
aattagtcag caaccatagt cccgccccta actccgccca tcccgcccct aactccgccc 4500
agttccgccc attctccgcc ccatggctga ctaatttttt ttatttatgc agaggccgag 4560
gccgcctcgg cctctgagct attccagaag tagtgaggag gcttttttgg aggcctaggc 4620
ttttgcaaaa agcttcacgc tgccgcaagc actcagggcg caagggctgc taaaggaagc 4680
ggaacacgta gaaagccagt ccgcagaaac ggtgctgacc ccggatgaat gtcagctact 4740
gggctatctg gacaagggaa aacgcaagcg caaagagaaa gcaggtagct tgcagtgggc 4800
ttacatggcg atagctagac tgggcggttt tatggacagc aagcgaaccg gaattgccag 4860
ctggggcgcc ctctggtaag gttgggaagc cctgcaaagt aaactggatg gctttcttgc 4920
cgccaaggat ctgatggcgc aggggatcaa gatctgatca agagacagga tgaggatcgt 4980
ttcgcatgat tgaacaagat ggattgcacg caggttctcc ggccgcttgg gtggagaggc 5040
tattcggcta tgactgggca caacagacaa tcggctgctc tgatgccgcc gtgttccggc 5100
tgtcagcgca ggggcgcccg gttctttttg tcaagaccga cctgtccggt gccctgaatg 5160
aactgcagga cgaggcagcg cggctatcgt ggctggccac gacgggcgtt ccttgcgcag 5220
ctgtgctcga cgttgtcact gaagcgggaa gggactggct gctattgggc gaagtgccgg 5280
ggcaggatct cctgtcatct caccttgctc ctgccgagaa agtatccatc atggctgatg 5340
caatgcggcg gctgcatacg cttgatccgg ctacctgccc attcgaccac caagcgaaac 5400
atcgcatcga gcgagcacgt actcggatgg aagccggtct tgtcgatcag gatgatctgg 5460
acgaagagca tcaggggctc gcgccagccg aactgttcgc caggctcaag gcgcgcatgc 5520
ccgacggcga ggatctcgtc gtgacccatg gcgatgcctg cttgccgaat atcatggtgg 5580
aaaatggccg cttttctgga ttcatcgact gtggccggct gggtgtggcg gaccgctatc 5640
aggacatagc gttggctacc cgtgatattg ctgaagagct tggcggcgaa tgggctgacc 5700
gcttcctcgt gctttacggt atcgccgctc ccgattcgca gcgcatcgcc ttctatcgcc 5760
ttcttgacga gttcttctga gcgggactct ggggttcgaa tcctaccagg gattggctga 5820
ctgatgagtc cgtgaggacg aaacgaaaaa catatggtac                       5860
```

<210> 6
<211> 4610

<212> DNA
<213> Influenza virus

<400> 6
gaggcatttc agtcagttgc tcaaggtacc aaaatgaaca ctcaaatcct ggttttcgcc 60
cttgcggcag tcatccccac aaatgcagac aaaatttgtc ttggacatca tgctgtatca 120
aatggcacca aagtaaacac actcactgag agaggagtag aagttgtcaa tgcaacggaa 180
acagtggagc ggacaaacat ccccaaaatt tgctcaaaag ggaaaagaac cactgatctt 240
ggccaatgcg gactgttagg gaccattacc ggaccacctc aatgcgacca atttctagaa 300
ttttcagctg atctaataat cgagagacga gaaggaaatg atgtttgtta cccggggaag 360
tttgttaatg aagaggcatt gcgacaaatc ctcagaggat caggtgggat tgacaaagaa 420
acaatgggat tcacatatag tggaataagg accaacggaa caactagtgc atgtagaaga 480
tcagggtctt cattctatgc agaaatggag tggctcctgt caaatacaga caatgcttct 540
ttcccacaaa tgacaaaatc atacaaaaac acagggagag aatcagctct gatagtctgg 600
ggaatccacc attcaggatc aaccaccgaa cagaccaaac tatatgggag tggaaataaa 660
ctgataacag tcgggagttc caaatatcat caatcttttg tgccgagtcc aggaacacga 720
ccgcagataa atggccggtc cggacggatt gattttcatt ggttgatctt ggatcccaat 780
gatacagtta cttttagttt caatggggct ttcatagctc caaatcgtgc cagcttcttg 840
aggggaaagt ccatggggat ccagagcgat gtgcaggttg atgctaattg cgaaggggaa 900
tgctaccaca gtggagggac tataacaagc agattgcctt ttcaaaacat aaatagcaga 960
gcagttggca aatgcccaag atatgtaaaa caggaaagtt tattattggc aactgggatg 1020
aagaacgttc ccgaaccttc caaaaaaagg aaaaaaagag gcctgtttgg tgctatagca 1080
gggtttattg aaaatggttg ggaaggtctg gtcgacgggt ggtacggttt caggcatcag 1140
aatgcacaag gagaaggaac tgcagcagac tacaaaagca cccaatcggc aattgatcag 1200
ataaccggaa agttaaatag actcattaag aaaaccaacc agcaatttga gctaatagat 1260
aatgaattca ctgaagtgga aaagcagatt ggcaatttaa ttaactggac caaagactcc 1320
atcacagaag tatggtctta caatgctgaa cttcttgtgg caatggaaaa ccagcacact 1380
attgatttgg ctgattcaga gatgaacaag ctgtatgagc gagtgaggaa acaattaagg 1440
gaaaatgctg aagaggatgg cactggttgc tttgaaattt ttcataaatg tgacgatgat 1500
tgtatggcta gtataaggaa caatacttat gatcacagca aatacagaga agaagcgatg 1560
caaaatagaa tacaaattga cccagtcaaa ttgagtagtg gctacaaaga tgtgatactt 1620
tggtttagct tcggggcatc atgctttttg cttcttgcca ttgcaatggg ccttgttttc 1680
atatgtgtga agaacggaaa catgcggtgc actatttgta tataggtttg gaaaaaaaca 1740
ccccttgttt ctactccccc ccaacttcgg aggtcgacca gtactccggg cgaaactttg 1800
tttttttttt ttcccccgat gctggaggtc gaccagatgt ccgaaagtgt cccccccccc 1860
cccccccccc ggcgcggaac ggcggggcca ctctggactc tttttttttt tttttttttt 1920
ttttttgggg atcggccgct agcttctgtt ttggcggatg agagaagatt ttcagcctga 1980
tacagattaa atcagaacgc agaagcggtc tgataaaaca gaatttgcct ggcggcagta 2040
gcgcggtggt cccacctgac cccatgccga actcagaagt gaaacgccgt agcgccgatg 2100
gtagtgtggg gtctccccat gcgagagtag ggaactgcca ggcatcaaat aaaacgaaag 2160
gctcagtcga aagactgggc ctttcgtttt atctgttgtt tgtcggtgaa cgctctcctg 2220
agtaggacaa atccgccggg agcggatttg aacgttgcga agcaacggcc cggagggtgg 2280
cgggcaggac gcccgccata aactgccagg catcaaatta agcagaaggc catcctgacg 2340
gatggccttt ttgcgtttct acaaactctt ttgtttattt ttctaaatac attcaaatat 2400
gtatccgctc atgagacaat aaccctgata aatgcttcaa taatattgaa aaaggaagag 2460
tatgagtatt caacatttcc gtgtcgccct tattcccttt tttgcggcat tttgccttcc 2520
tgtttttgct cacccagaaa cgctggtgaa agtaaaagat gctgaagatc agttgggtgc 2580
acgagtgggt tacatcgaac tggatctcaa cagcggtaag atccttgaga gttttcgccc 2640
cgaagaacgt tttccaatga tgagcacttt taaagttctg ctatgtggcg cggtattatc 2700
ccgtgttgac gccgggcaag agcaactcgg tcgccgcata cactattctc agaatgactt 2760
ggttgagtac tcaccagtca cagaaaagca tcttacggat ggcatgacag taagagaatt 2820
atgcagtgct gccataacca tgagtgataa cactgcggcc aacttacttc tgacaacgat 2880
cggaggaccg aaggagctaa ccgctttttt gcacaacatg ggggatcatg taactcgcct 2940
tgatcgttgg gaaccggagc tgaatgaagc cataccaaac gacgagcgtg acaccacgat 3000
gcctgtagca atggcaacaa cgttgcgcaa actattaact ggcgaactac ttactctagc 3060

ttcccggcaa caattaatag actggatgga ggcggataaa gttgcaggac cacttctgcg 3120
ctcggccctt ccggctggct ggtttattgc tgataaatct ggagccggtg agcgtgggtc 3180
tcgcggtatc attgcagcac tggggccaga tggtaagccc tcccgtatcg tagttatcta 3240
cacgacgggg agtcaggcaa ctatggatga acgaaataga cagatcgctg agataggtgc 3300
ctcactgatt aagcattggt aactgtcaga ccaagtttac tcatatatac tttagattga 3360
tttaaaactt catttttaat ttaaaaggat ctaggtgaag atcctttttg ataatctcat 3420
gaccaaaatc ccttaacgtg agttttcgtt ccactgagcg tcagaccccg tagaaaagat 3480
caaaggatct tcttgagatc ctttttttct gcgcgtaatc tgctgcttgc aaacaaaaaa 3540
accaccgcta ccagcggtgg tttgtttgcc ggatcaagag ctaccaactc tttttccgaa 3600
ggtaactggc ttcagcagag cgcagatacc aaatactgtc cttctagtgt agccgtagtt 3660
aggccaccac ttcaagaact ctgtagcacc gcctacatac ctcgctctgc taatcctgtt 3720
accagtggct gctgccagtg gcgataagtc gtgtcttacc gggttggact caagacgata 3780
gttaccggat aaggcgcagc ggtcgggctg aacggggggt tcgtgcacac agcccagctt 3840
ggagcgaacg acctacaccg aactgagata cctacagcgt gagctatgag aaagcgccac 3900
gcttcccgaa gggagaaagg cggacaggta tccggtaagc ggcagggtcg gaacaggaga 3960
gcgcacgagg gagcttccag ggggaaacgc ctggtatctt tatagtcctg tcgggtttcg 4020
ccacctctga cttgagcgtc gatttttgtg atgctcgtca ggggggcgga gcctatggaa 4080
aaacgccagc aacgcggcct ttttacggtt cctggccttt tgctggcctt ttgctcacat 4140
gttctttcct gcgttatccc ctgattcatt aatgcaggtc acgatccttt ctggcgagtc 4200
cccgtgcgga gtcggagagc gctccctgag cgcgtgcggc ccgagaggtc gcgcctggcc 4260
ggccttcggt ccctcgtgtg tcccggtcgt aggaggggcc ggccgaaaat gcttccggct 4320
cccgctctgg agacacgggc cggccccctg cgtgtggcac gggcggccgg gaggcgtcc 4380
ccggcccggc gctgctcccg cgtgtgtcct ggggttgacc agagggcccc gggcgctccg 4440
tgtgtggctg cgatggtggc gtttttgggg acaggtgtcc gtgtccgtgt cgcgcgtcgc 4500
ctgggccggc ggcgtggtcg gtgacgcgac ctcccggccc cgggggaggt atatctttcg 4560
ctccgagtcg gcattttggg ccgccgggtt attagtagaa acaggggtac 4610

<210> 7
<211> 3558
<212> DNA
<213> Influenza virus

<400> 7
tattagtaga aacagggtat tttttattct agtacattac gccccgccct gccactcatc 60
gcagtactgt tgtaattcat taagcattct gccgacatgg aagccatcac agacggcatg 120
atgaacctga atcgccagcg gcatcagcac cttgtcgcct tgcgtataat atttgcccat 180
ggtgaaaacg ggggcgaaga agttgtccat attggccacg tttaaatcaa aactggtgaa 240
actcacccag ggattggctg agacgaaaaa catattctca ataaaccctt tagggaaata 300
ggccaggttt tcaccgtaac acgccacatc ttgcgaatat atgtgtagaa actgccggaa 360
atcgtcgtgg tattcactcc agagcgatga aaacgtttca gtttgctcat ggaaaacggt 420
gtaacaaggg tgaacactat cccatatcac cagctcaccg tctttcattg ccatacggaa 480
ttccggatga gcattcatca ggcgggcaag aatgtgaata aaggccggat aaaacttgtg 540
cttatttttc tttacggtct ttaaaaaggc cgtaatatcc agctgaacgg tctggttata 600
ggtacattga gcaactgact gaaatgcctc aaaatgttct ttacgatgcc attgggatat 660
atcaacggtg gtatatccag tgattttttt ctccatgatt atggccatta cccttgtttc 720
tactcccccc caacttcgga ggtcgaccag tactccgggc gaaactttgt ttttttttt 780
tcccccgatg ctggaggtcg accagatgtc cgaaagtgtc cccccccccc ccccccccg 840
gcgcggaacg gcggggccac tctggactct ttttttttt ttttttttt tttttgggga 900
tcggccgcta gcttctgttt tggcggatga gagaagattt tcagcctgat acagattaaa 960
tcagaacgca gaagcggtct gataaaacag aatttgcctg gcggcagtag cgcggtggtc 1020
ccacctgacc ccatgccgaa ctcagaagtg aaacgccgta gcgccgatgg tagtgtgggg 1080
tctccccatg cgagagtagg gaactgccag gcatcaaata aaacgaaagg ctcagtcgaa 1140
agactgggcc tttcgtttta tctgttgttt gtcggtgaac gctctcctga gtaggacaaa 1200
tccgccggga gcggatttga acgttgcgaa gcaacggccc ggagggtggc gggcaggacg 1260
cccgccataa actgccaggc atcaaattaa gcagaaggcc atcctgacgg atggcctttt 1320

tgcgtttcta caaactcttt tgtttatttt tctaaataca ttcaaatatg tatccgctca 1380
tgagacaata accctgataa atgcttcaat aatattgaaa aaggaagagt atgagtattc 1440
aacatttccg tgtcgccctt attccctttt ttgcggcatt ttgccttcct gtttttgctc 1500
acccagaaac gctggtgaaa gtaaaagatg ctgaagatca gttgggtgca cgagtgggtt 1560
acatcgaact ggatctcaac agcggtaaga tccttgagag ttttcgcccc gaagaacgtt 1620
ttccaatgat gagcactttt aaagttctgc tatgtggcgc ggtattatcc cgtgttgacg 1680
ccgggcaaga gcaactcggt cgccgcatac actattctca gaatgacttg gttgagtact 1740
caccagtcac agaaaagcat cttacggatg gcatgacagt aagagaatta tgcagtgctg 1800
ccataaccat gagtgataac actgcggcca acttacttct gacaacgatc ggaggaccga 1860
aggagctaac cgcttttttg cacaacatgg gggatcatgt aactcgcctt gatcgttggg 1920
aaccggagct gaatgaagcc ataccaaacg acgagcgtga caccacgatg cctgtagcaa 1980
tggcaacaac gttgcgcaaa ctattaactg gcgaactact tactctagct tcccggcaac 2040
aattaataga ctggatggag gcggataaag ttgcaggacc acttctgcgc tcggcccttc 2100
cggctggctg gtttattgct gataaatctg gagccggtga gcgtgggtct cgcggtatca 2160
ttgcagcact ggggccagat ggtaagccct cccgtatcgt agttatctac acgacgggga 2220
gtcaggcaac tatggatgaa cgaaatagac agatcgctga gataggtgcc tcactgatta 2280
agcattggta actgtcagac caagtttact catatatact ttagattgat ttaaaacttc 2340
atttttaatt taaaaggatc taggtgaaga tcctttttga taatctcatg accaaaatcc 2400
cttaacgtga gttttcgttc cactgagcgt cagaccccgt agaaaagatc aaaggatctt 2460
cttgagatcc tttttttctg cgcgtaatct gctgcttgca aacaaaaaaa ccaccgctac 2520
cagcggtggt ttgtttgccg gatcaagagc taccaactct ttttccgaag gtaactggct 2580
tcagcagagc gcagatacca aatactgtcc ttctagtgta gccgtagtta ggccaccact 2640
tcaagaactc tgtagcaccg cctacatacc tcgctctgct aatcctgtta ccagtggctg 2700
ctgccagtgg cgataagtcg tgtcttaccg ggttggactc aagacgatag ttaccggata 2760
aggcgcagcg gtcgggctga acggggggtt cgtgcacaca gcccagcttg gagcgaacga 2820
cctacaccga actgagatac ctacagcgtg agctatgaga aagcgccacg cttcccgaag 2880
ggagaaaggc ggacaggtat ccggtaagcg gcagggtcgg aacaggagag cgcacgaggg 2940
agcttccagg gggaaacgcc tggtatcttt atagtcctgt cgggtttcgc cacctctgac 3000
ttgagcgtcg atttttgtga tgctcgtcag gggggcggag cctatggaaa aacgccagca 3060
acgcggcctt tttacggttc ctggcctttt gctggccttt tgctcacatg ttctttcctg 3120
cgttatcccc tgattcatta atgcaggtca cgatcctttc tggcgagtcc ccgtgcggag 3180
tcggagagcg ctccctgagc gcgtgcggcc cgagaggtcg cgcctggccg gccttcggtc 3240
cctcgtgtgt cccggtcgta ggaggggccg gccgaaaatg cttccggctc ccgctctgga 3300
gacacgggcc ggccccctgc gtgtggcacg ggcggccggg agggcgtccc cggcccggcg 3360
ctgctcccgc gtgtgtcctg ggttgacca gagggccccg ggcgctccgt gtgtggctgc 3420
gatggtggcg tttttgggga caggtgtccg tgtccgtgtc gcgcgtcgcc tgggccggcg 3480
gcgtggtcgg tgacgcgacc tcccggcccc gggggaggta tatctttcgc tccgagtcgg 3540
cattttgggc cgccgggt 3558

<210> 8
<211> 4343
<212> DNA
<213> Influenza virus

<400> 8
ctttctggcg agtccccgtg cggagtcgga gagcgctccc tgagcgcgtg cggcccgaga 60
ggtcgcgcct ggccggcctt cggtccctcg tgtgtcccgg tcgtaggagg ggccggccga 120
aaatgcttcc ggctcccgct ctggagacac gggccggccc cctgcgtgtg gcacgggcgg 180
ccgggagggc gtccccggcc cggcgctgct cccgcgtgtg tcctggggtt gaccagaggg 240
ccccgggcgc tccgtgtgtg gctgcgatgg tggcgttttt ggggacaggt gtccgtgtcc 300
gtgtcgcgcg tcgcctgggc cggcggcgtg gtcggtgacg cgacctcccg gccccggggg 360
aggtatatct ttcgctccga gtcggcattt tgggccgccg ggttattagt agaaacaggg 420
tatttttat actagtaagc tcgaaggagt ccaccatgag taaaggagaa gaacttttca 480
ctggagttgt cccaattctt gttgaattag atggtgatgt taatgggcac aaattttctg 540
tcagtggaga gggtgaaggt gatgcaacat acggaaaact tacccttaaa tttatttgca 600

ctactggaaa actacctgtt ccatggccaa cacttgtcac tactttcact tatggtgttc 660
aatgcttttc aagataccca gatcatatga aacagcatga ctttttcaag agtgccatgc 720
ccgaaggtta tgtacaggaa agaactatat ttttcaaaga tgacgggaac tacaagacac 780
gtgctgaagt caagtttgaa ggtgataccc ttgttaatag aatcgagtta aaaggtattg 840
attttaaaga agatggaaac attcttggac acaaattgga atacaactat aactcacaca 900
atgtatacat catggctgac aagcagaaga acggaatcaa ggccaacttc aagacccgcc 960
acaacatcga ggacggcggc gtgcagctgg ccgaccacta ccagcagaac accccaattg 1020
gcgatggccc tgtcctttta ccagacaacc attacctgtc cacacaatct gccctttcga 1080
aagatcccaa cgaaaagaga gaccacatgg tccttcttga gtttgtaaca gctgctggga 1140
ttacacatgg catggatgaa ctatacaagg gatcccatca ccatcaccat cactaagctc 1200
catggtctag atatctagta cattacgccc cgccctgcca ctcatcgcag tactgttgta 1260
attcattaag cattctgccg acatggaagc catcacagac ggcatgatga acctgaatcg 1320
ccagcggcat cagcaccttg tcgccttgcg tataatattt gcccatggtg aaaacggggg 1380
cgaagaagtt gtccatattg gccacgttta aatcaaaact ggtgaaactc acccagggat 1440
tggcactcac aaagaacatg ttctcgatga atcctttagg gaagtaggcc aggttttcac 1500
cgtaacacgc cacatcttgc gaatatatgt gtagaaactg ccggaaatcg tcgtggtatt 1560
cactccagag cgatgaaaac gtttcagttt gctcatggaa aacggtgtaa caagggtgaa 1620
cactatccca tatcaccagc tcaccgtctt tcattgccat acggaattcc ggatgagcat 1680
tcatcaggcg ggcaagaatg tgaataaagg ccggataaaa cttgtgctta tttttcttta 1740
cggtctttaa aaaggccgta atatccagct gaacggtctg gttataggta cattgagcaa 1800
ctgactgaaa tgcctcaaaa tgttctttac gatgccattg ggatatatca acggtggtat 1860
atccagtgat ttttttctcc atgattatgc aaaaaatacc cttgtttcta ctcccccccca 1920
acttcggagg tcgaccagta ctccgggcga aactttgttt tttttttttc ccccgatgct 1980
ggaggtcgac cagatgtccg aaagtgtccc cccccccccc ccccccccggc gcggaacggc 2040
ggggccactc tggactcttt tttttttttt tttttttttt tttggggatc ggccgctagc 2100
ttctgttttg gcggatgaga gaagattttc agcctgatac agattaaatc agaacgcaga 2160
agcggtctga taaaacagaa tttgcctggc ggcagtagcg cggtggtccc acctgacccc 2220
atgccgaact cagaagtgaa acgccgtagc gccgatggta gtgtggggtc tccccatgcg 2280
agagtaggga actgccaggc atcaaataaa acgaaaggct cagtcgaaag actgggcctt 2340
tcgttttatc tgttgtttgt cggtgaacgc tctcctgagt aggacaaatc cgccgggagc 2400
ggatttgaac gttgcgaagc aacggcccgg agggtggcgg gcaggacgcc cgccataaac 2460
tgccaggcat caaattaagc agaaggccat cctgacggat ggcctttttg cgtttctaca 2520
aactcttttg tttatttttc taaatacatt caaatatgta tccgctcatg agacaataac 2580
cctgataaat gcttcaataa tattgaaaaa ggaagagtat gagtattcaa catttccgtg 2640
tcgcccttat tccctttttt gcggcatttt gccttcctgt ttttgctcac ccagaaacgc 2700
tggtgaaagt aaaagatgct gaagatcagt tgggtgcacg agtgggttac atcgaactgg 2760
atctcaacag cggtaagatc cttgagagtt ttcgccccga agaacgtttt ccaatgatga 2820
gcacttttaa agttctgcta tgtggcgcgg tattatcccg tgttgacgcc gggcaagagc 2880
aactcggtcg ccgcatacac tattctcaga atgacttggt tgagtactca ccagtcacag 2940
aaaagcatct tacggatggc atgacagtaa gagaattatg cagtgctgcc ataaccatga 3000
gtgataacac tgcggccaac ttacttctga caacgatcgg aggaccgaag gagctaaccg 3060
cttttttgca caacatgggg gatcatgtaa ctcgccttga tcgttgggaa ccggagctga 3120
atgaagccat accaaacgac gagcgtgaca ccacgatgcc tgtagcaatg gcaacaacgt 3180
tgcgcaaact attaactggc gaactactta ctctagcttc ccggcaacaa ttaatagact 3240
ggatggaggc ggataaagtt gcaggaccac ttctgcgctc ggcccttccg gctggctggt 3300
ttattgctga taaatctgga gccggtgagc gtgggtctcg cggtatcatt gcagcactgg 3360
ggccagatgg taagccctcc cgtatcgtag ttatctacac gacggggagt caggcaacta 3420
tggatgaacg aaatagacag atcgctgaga taggtgcctc actgattaag cattggtaac 3480
tgtcagacca agtttactca tatatacttt agattgattt aaaacttcat ttttaattta 3540
aaaggatcta ggtgaagatc ctttttgata atctcatgac caaaatccct taacgtgagt 3600
tttcgttcca ctgagcgtca gaccccgtag aaaagatcaa aggatcttct tgagatcctt 3660
tttttctgcg cgtaatctgc tgcttgcaaa caaaaaaacc accgctacca gcggtggttt 3720
gtttgccgga tcaagagcta ccaactcttt ttccgaaggt aactggcttc agcagagcgc 3780
agataccaaa tactgtcctt ctagtgtagc cgtagttagg ccaccacttc aagaactctg 3840
tagcaccgcc tacatacctc gctctgctaa tcctgttacc agtggctgct gccagtggcg 3900

```
ataagtcgtg tcttaccggg ttggactcaa gacgatagtt accggataag gcgcagcggt 3960
cgggctgaac ggggggttcg tgcacacagc ccagcttgga gcgaacgacc tacaccgaac 4020
tgagatacct acagcgtgag ctatgagaaa gcgccacgct tcccgaaggg agaaaggcgg 4080
acaggtatcc ggtaagcggc agggtcggaa caggagagcg cacgagggag cttccagggg 4140
gaaacgcctg gtatctttat agtcctgtcg ggtttcgcca cctctgactt gagcgtcgat 4200
ttttgtgatg ctcgtcaggg gggcggagcc tatggaaaaa cgccagcaac gcggcctttt 4260
tacggttcct ggccttttgc tggccttttg ctcacatgtt ctttcctgcg ttatcccctg 4320
attcattaat gcaggtcacg atc                                         4343
```

<210> 9
<211> 3888
<212> DNA
<213> Influenza virus

<400> 9

```
cccaaaaaaa aaaaaaaaaa aaaaaaaaag agtccagagt ggccccgccg ttccgcgccg 60
gggggggggg gggggggga cactttcgga catctggtcg acctccagca tcgggggaaa 120
aaaaaaaaac aaagtttcgc ccggagtact ggtcgacctc cgaagttggg ggggagtaga 180
aacagggtag ataatcactc actgagtgac atccacatcg cgagcgcgcg taatacgact 240
cactataggg cgaattgggt accgggcccc ccctcgaggt cgacggtatc gataagcttc 300
gacgagattt tcaggagcta aggaagctaa aatggagaaa aaaatcactg gatataccac 360
cgttgatata tcccaatggc atcgtaaaga acattttgag gcatttcagt cagttgctca 420
atgtacctat aaccagaccg ttcagctgga tattacggcc tttttaaaga ccgtaaagaa 480
aaataagcac aagttttatc cggcctttat tcacattctt gcccgcctga tgaatgctca 540
tccggaattc cgtatggcaa tgaaagacgg tgagctggtg atatgggata gtgttcaccc 600
ttgttacacc gttttccatg agcaaactga aacgttttca tcgctctgga gtgaatacca 660
cgacgatttc cggcagtttc tacacatata ttcgcaagat gtggcgtgtt acggtgaaaa 720
cctggcctat ttccctaaag ggtttattga gaatatgttt ttcgtctcag ccaatccctg 780
ggtgagtttc accagttttg atttaaacgt ggccaatatg gacaacttct tcgcccccgt 840
tttcaccatg ggcaaatatt atacgcaagg cgacaaggtg ctgatgccgc tggcgattca 900
ggttcatcat gccgtttgtg atggcttcca tgtcggcaga atgcttaatg aattacaaca 960
gtactgcgat gagtggcagg gcggggcgta atttttttaa ggcagttatt ggtgccctta 1020
aacgcctggt gctacgcctg aataagtgat aataagcgga tgaatggcag aaattcgtcg 1080
aagcttgata tcgaattcct gcagcccggg ggatccacta gttctagagc ggccgccacc 1140
gcggtggagc tccagctttt gttcccttta gtgagggtta attgcgcgca ggcctagcta 1200
ggtaaagaaa aataccttg attcttctaa taacccggcg gcccaaaatg ccgactcgga 1260
gcgaaagata tacctccccc ggggccggga ggtcgcgtca ccgaccacgc cgccggccca 1320
ggcgacgcgc gacacggaca cctgtcccca aaaacgccac catcgcagcc acacacggag 1380
cgcccggggc cctctggtca accccaggac acacgcggga gcagcgccgg gccgggggacg 1440
ccctcccggc cgcccgtgcc acacgcaggg ggccggcccg tgtctccaga gcgggagccg 1500
gaagcatttt cggccggccc ctcctacgac cgggacacac gaggaccgga aggccggcca 1560
ggcgcgacct ctcgggccgc acgcgcgctc agggagcgct ctccgactcc gcacggggac 1620
tcgccagaaa ggatcgtgac ctgcattaat gaatcagggg ataacgcagg aaagaacatg 1680
tgagcaaaag gccagcaaaa ggccaggaac cgtaaaaagg ccgcgttgct ggcgtttttc 1740
cataggctcc gcccccctga cgagcatcac aaaaatcgac gctcaagtca gaggtggcga 1800
aacccgacag gactataaag ataccaggcg tttccccctg gaagctccct cgtgcgctct 1860
cctgttccga ccctgccgct taccggatac ctgtccgcct ttctcccttc gggaagcgtg 1920
gcgctttctc atagctcacg ctgtaggtat ctcagttcgg tgtaggtcgt tcgctccaag 1980
ctgggctgtg tgcacgaacc ccccgttcag cccgaccgct gcgccttatc cggtaactat 2040
cgtcttgagt ccaacccggt aagacacgac ttatcgccac tggcagcagc cactggtaac 2100
aggattagca gagcgaggta tgtaggcggt gctacagagt tcttgaagtg gtggcctaac 2160
tacggctaca ctagaaggac agtatttggt atctgcgctc tgctgaagcc agttaccttc 2220
ggaaaaagag ttggtagctc ttgatccggc aaacaaacca ccgctggtag cggtggtttt 2280
tttgtttgca agcagcagat tacgcgcaga aaaaaaggat ctcaagaaga tcctttgatc 2340
ttttctacgg ggtctgacgc tcagtggaac gaaaactcac gttaagggat tttggtcatg 2400
```

```
agattatcaa aaaggatctt cacctagatc cttttaaatt aaaaatgaag ttttaaatca 2460
atctaaagta tatatgagta aacttggtct gacagttacc aatgcttaat cagtgaggca 2520
cctatctcag cgatctgtct atttcgttca tccatagttg cctgactccc cgtcgtgtag 2580
ataactacga tacgggaggg cttaccatct ggccccagtg ctgcaatgat accgcgagac 2640
ccacgctcac cggctccaga tttatcagca ataaaccagc cagccggaag ggccgagcgc 2700
agaagtggtc ctgcaacttt atccgcctcc atccagtcta ttaattgttg ccgggaagct 2760
agagtaagta gttcgccagt taatagtttg cgcaacgttg ttgccattgc tacaggcatc 2820
gtggtgtcac gctcgtcgtt tggtatggct tcattcagct ccggttccca acgatcaagg 2880
cgagttacat gatcccccat gttgtgcaaa aaagcggtta gctccttcgg tcctccgatc 2940
gttgtcagaa gtaagttggc cgcagtgtta tcactcatgg ttatggcagc actgcataat 3000
tctcttactg tcatgccatc cgtaagatgc ttttctgtga ctggtgagta ctcaaccaag 3060
tcattctgag aatagtgtat gcggcgaccg agttgctctt gcccggcgtc aacacgggat 3120
aataccgcgc cacatagcag aactttaaaa gtgctcatca ttggaaaacg ttcttcgggg 3180
cgaaaactct caaggatctt accgctgttg agatccagtt cgatgtaacc cactcgtgca 3240
cccaactgat cttcagcatc ttttactttc accagcgttt ctgggtgagc aaaaacagga 3300
aggcaaaatg ccgcaaaaaa gggaataagg gcgacacgga aatgttgaat actcatactc 3360
ttcctttttc aatattattg aagcatttat cagggttatt gtctcatgag cggatacata 3420
tttgaatgta tttagaaaaa taaacaaaag agtttgtaga aacgcaaaaa ggccatccgt 3480
caggatggcc ttctgcttaa tttgatgcct ggcagtttat ggcgggcgtc ctgcccgcca 3540
ccctccgggc cgttgcttcg caacgttcaa atccgctccc ggcggatttg tcctactcag 3600
gagagcgttc accgacaaac aacagataaa acgaaaggcc cagtctttcg actgagcctt 3660
tcgttttatt tgatgcctgg cagttcccta ctctcgcatg gggagacccc acactaccat 3720
cggcgctacg gcgtttcact tctgagttcg gcatggggtc aggtgggacc accgcgctac 3780
tgccgccagg caaattctgt tttatcagac cgcttctgcg ttctgattta atctgtatca 3840
ggctgaaaat cttctctcat ccgccaaaac agaagctagc ggccgatc              3888
```

## Description

The invention relates to a recombinant influenza virus for high-yield expression of incorporated foreign gene (s), which is genetically stable in the absence of any helper virus; a method for the production of said recombinant influenza virus, a method for constructing specific ribozyme-sensitive influenza carrier strains; pharmaceutical compositions comprising said recombinant influenza viruses; and the use of said recombinant influenza virus for preparing medicaments for vaccination purposes.

Redesigning influenza virus into a vector system for expression of foreign genes similar to what has been achieved in several other thoroughly studied viruses such as adenovirus, retrovirus, Semliki Forest virus or Rabies virus has the advantage of an industrially well established mode of cheap propagation for influenza in fertilized chicken eggs leading to rather high titers (above $10^{10}$/ml). On the other hand none of its genes may be deleted from the influenza genome according to our present knowledge, and give room for large-size foreign insertions. Only small fragments of foreign polypeptide chains such as B cell epitopes (10 to 15 amino acids) may be inserted into selected positions within two of the viral proteins, i.e. in exchange for one of the variable antigenic regions located at the surface of hemagglutinin (Muster et al., Mucosal model of immunization against human immunodeficiency virus type 1 with a chimeric influenza virus. J. Virol. 69 (11), 6678-6686 (1995)), or into the stalk sequence of viral neuraminidase (Garcia-Sastre and Palese, The cytoplasmic tail of the neuraminidase protein of influenza A virus does not play an important role in the packaging of this protein into viral envelopes. Virus Res. 37, 37-47 (1995)), and be stably maintained as functional fusion proteins. Constructs of this kind turned out to be useful for experimental vaccination in a few cases studied, but only rather few clearly defined epitope sequences (of ten to twelve amino acids each) are known today, and some of them might also be misfolded within such restricted fusion protein positions, or in other cases interfere with the correct tertiary structure and function of their host polypeptide chains.

Incorporation of a full-size foreign protein into influenza via reverse genetics, encoded by an independent ninth vRNA molecule in addition to its regular set of eight standard vRNA segments is without special provisions only transiently possible (Luytjes et al., Amplification, expression, and packaging of a foreign gene by influenza virus. Cell 59, 1107-1113 (1989); Enami et al., An influenza virus containing nine different RNA segments. Virology 185, 291-298 (1991)). In the absence of a continuous selective pressure any additional recombinant vRNA segment cannot be stably maintained as long as a wildtype promoter sequence is used on that ninth vRNA segment, and it will inadvertently be lost after few steps of propagation. Using a different system of influenza reverse genetics developed in our laboratory (Zobel et al., RNA polymerase I catalysed transcription of insert viral cDNA. Nucleic Acids Res. 21, 3607-3614 (1993); Neumann et al., RNA polymerase I-mediated expression of influenza viral RNA molecules. Virology 202, 477-479 (1994)), which was built around *in vivo* synthesis of recombinant vRNA molecules by cellular RNA polymerase I transcription of the respective template cDNA constructs, promoter-up mutations have been designed by nucleotide substitutions (Neumann and Hobom, Mutational analysis of influenza virus promoter elements *in vivo.* J. Gen. Virol. 76, 1709-1717 (1995)). When these are attached to a recombinant ninth vRNA segment its increased transcription and amplification rate will not only compensate for the losses suffered spontaneously, but even cause accumulation of the foreign vRNA segment during simple viral passaging, in the absence of any selection. However, due to its over-replication relative to all of the regular influenza vRNA segments (which of course are connected to wild-type promoter sequences) after catching up with the others the foreign segment will become over-abundant. This increasingly will result in viral particles that have incorporated several copies of recombinant vRNA, but no longer have a full set of all eight standard segments incorporated among an average of about 15 vRNA molecules present within a virion. Such particles are defective and will not result in plaque formation, hence after an initial increase of recombinant viral particles during the first steps of propagation a dramatic decrease is observed, usually at the third or fourth step of viral passaging, depending on the size of the recombinant vRNA and the level of the promoter-up mutation attached. A balanced situation with regard to the insert length and the level of promoter activity can be achieved, and has been propagated in a particular case over 11 passages, with essentially stable levels of recombinant viruses among a majority of helper viruses (around 80%) during these steps. If a full-level promoter-up mutation is used (1104 or the new variant 1920, see below) a balanced-level propagation is reached in conjunction with a recombinant vRNA size of 4000 nucleotides (unpublished).

In all of these preparations, both in transiently achieved increased yields (up to 40% of recombinants after three or four steps of viral passage), and in a balanced propagation of recombinant influenza viruses (10 - 20%) the respective viral progeny inadvertantly constitute mixtures with a majority of non-recombinant helper viruses. These result both from a statistical mode of packaging vRNA molecules into a virion (the ninth segment may not be co-packaged), and from the fraction of cells solely infected by helper virus. The problems of fractional yields and of instability during viral propagation of recombinant influenza are the problems to be solved with the present invention.

Starting out from two observations in this laboratory which are discussed below and which concern two hitherto unsuspected properties of influenza viral RNA polymerase in its interaction with terminally adapted influenza-specific RNA molecules, a new technique for the construction of stable recombinant influenza viruses was found.

As previously described in WO 96/10641 plasmid constructs are designed to generate influenza vRNA-like molecules *in vivo* by cellular RNA polymerase I transcription following plasmid DNA transfection into tissue culture cells, and to this end contain flanking rDNA promoter and terminator elements, externally located relative to any cDNA constructs in between. The resulting recombinant vRNA molecules are designed to contain 5' and 3' recognition sequences for influenza viral RNA polymerase, which however carry up to five nucleotide substitutions (in promoter-up mutant pHL1920) resulting in a substantial increase of expression over wildtype influenza promoter levels. While recombinant pseudoviral RNA is initially transcribed by RNA polymerase I, further amplification and mRNA transcription depends on the presence of viral RNA polymerase and viral nucleoprotein in the cell, which generally are provided by infection of a helper virus. As a consequence the progeny viral yield will constitute a mixture of recombinant viruses together with a majority of wild-type helper viruses.

In the new technique the recombinant vRNA-like molecules as transcribed by RNA polymerase I are constructed as ambisense RNA double segments, with one reading frame (an influenza gene) in sense and a second one (a foreign gene) in anti-sense orientation, or vice versa. In such constructs both reading frames will be expressed via the cap-snatching mode, even if at different levels. Again, infection by helper virus is required to provide the necessary viral early and late proteins for genetic expression and virion packaging. However, the particular helper virus used in the new method is a recombinant virus carrying 2x2 specifically designed ribozyme targets inserted into the flanking non-coding sequences of one of its eight vRNA segments. The viral segment chosen to become ribozyme-sensitive is always identical to the one used in constructing the recombinant ambisense RNA molecule, as the viral carrier gene in covalent linkage with an additional foreign gene.

Recombinant influenza viruses produced in this way through RNA polymerase I transcription of an ambisense viral RNA molecule followed by infection with that specifically designed type of ribozyme-sensitive helper virus will carry one of the influenza genes twice, once within that ribozyme-sensitive helper vRNA segment, and a second time within the ribozyme-resistant ambisense segment. Recombinant viruses of this type are again obtained initially only as a mixture together with a majority of non-recombinant helper viruses. A progeny viral passage through tissue culture cells (293T) which before have been transiently transfected with plasmid constructs expressing the respective double-headed ribozyme will (in one step) inactivate the ribozyme-sensitive segment by a factor of up to 100. One or two rounds of such ribozyme treatment *in vivo* will at the same time (a) purify the recombinant virus from its non-recombinant helper contaminants, and (b) delete the sensitive vRNA helper segment from within the initial (additive) recombinant virus.

As a result recombinant influenza viruses are isolated along this several-step procedure, which are free of contaminating helper viruses and carry seven regular and one ambisense vRNA segments, all in a balanced replication mode. Their recombinant nature is stably maintained because of a covalent junction between one of the viral genes and the full-size foreign gene inserted, a situation achieved here for the first time, via constructing an influenza ambisense RNA segment. The whole procedure is independent of any (selectable) phenotype, and can be applied to either of the eight influenza vRNA segments. After establishing a first ambisense vRNA segment carrying a single foreign gene it can also be repeated all over for inserting a second foreign gene within another ambisense RNA segment of the same constitution in principle.

Stable recombinant viruses of the type described can be used for cheap propagation in fertilized eggs, either for production of those recombinant viruses themselves or for production of foreign proteins or glycoproteins encoded by them, and hence find application in (glyco)protein production or in providing vector systems for somatic gene therapy or in being used as vaccination agents.

Thus, the present invention provides

(1) a recombinant influenza virus for high-yield expression of incorporated foreign gene(s), which is genetically stable in the absence of any helper virus, wherein

(a) at least one of the regular viral RNA segments has been exchanged for a vRNA encoding a foreign gene; and/or

(b) at least one of the regular viral RNA segments is an ambisense RNA molecule, containing one of the standard viral genes in sense orientation and a foreign, recombinant gene in anti-sense orientation, or *vice versa,* in overall convergent arrangement;

(2) a method for the production of recombinant influenza viruses as defined in (1) above comprising

(a) RNA polymerase I synthesis of recombinant vRNAs *in vivo,* in antisense, sense or ambisense design,

(b) followed by infection with an influenza carrier strain constructed to include flanking ribozyme target sequences in at least one of its viral RNA segments, and

(c) thereafter selective vRNA inactivation through ribozyme cleavage;

(3) a method of constructing influenza carrier strains carrying one or more ribozyme target sites (of type one) in vRNA flanking positions comprising

(a) RNA polymerase I synthesis of recombinant vRNAs *in vivo,* carrying two different 3' promoter sequences in tandem, which are separated by a second type of ribozyme target sequence, and which carry the said internal ribozyme target sites of type one;
(b) followed by infection of an influenza wildtype strain;
(c) thereafter amplification through simple steps of viral propagation; and
(d) finally isolation through removal of their external 3' promoter sequence by ribozyme cleavage through infection of cells expressing ribozyme type 2, followed by plaque purification;
said method being suitable for the constuction of an influenza carrier strain required for step (b) of (2) above;

(4) a ribozyme-sensitive influenza carrier (helper) strain obtainable by the method of (3) above;
(5) a pharmaceutical composition comprising a recombinant virus as defined in (1) above;
(6) use of a recombinant virus as defined in (1) above for preparing a medicament for vaccination purposes;
(7) use of a recombinant virus as defined in (1) above as vector systems in somatic gene therapy, for transfer and expression of foreign genes into cells (abortively) infected by such viruses, either in ex *vivo* or *in vivo* application schemes; and
(8) a method for the production of proteins or glycoproteins which comprises utilizing a recombinant influenza virus as defined in (1) above as expression vector.

Moreover, the invention provides a method for preventing/treating influenza utilizing a recombinant virus as defined in (1) above, i.e., a vaccination method utilizing said recombinant virus.

According to the present invention "influenza virus" embraces influenza A virus, influenza B virus and influenza C virus. A preferred recombinant virus of the invention is where one or both of the standard glycoproteins hemagglutinin and neuraminidase have been exchanged into foreign glycoprotein(s) or into fusion glycoproteins consisting of an anchor segment derived from hemagglutinin and an ectodomain obtained from the foreign source, viral or cellular, or in which such recombinant glycoprotein has been inserted as a third molecular species in addition to the remaining standard components.

Further preferred recombinant viruses of the invention are where the terminal viral RNA sequences, which are active as the promoter signal, have been varied by nucleotide substitutions in up to five positions, resulting in improved transcription rates of both the vRNA promoter as well as the cRNA promoter as present in the complementary sequence.

Further preferred embodiments of the invention are set forth herein below.

<u>A. Construction of influenza helper virus strains carrying ribozyme target sequences in flanking positions within either of the vRNA segments</u>

A.1. Influenza RNA polymerase will initiate transcription and replication from promoter structures located at internal positions in an RNA molecule, not only from the natural position at both ends of a vRNA molecule:

This is true in particular for promoter-up variants in RNA-internal location due to their enhanced binding affinity for viral polymerase. Not only 3' end extensions are tolerated in RNA-internal promoter recognition (Fig. 1), but also 5' extensions as well as extensions at both ends of the RNA template molecule, containing noncomplementary as well as complementary sequence, i.e. potentially present as a double-stranded extension. Finally, also an extension by way of duplication of the promoter sequence (active or inactivated) leads to mRNA transcription and CAT expression, initiated from the active pair of 5' and 3' promoter halves, irrespective if in external or in internal or even in an oblique localization (active 5' promoter sequence in external, active 3' promoter sequence in internal position, or vice versa). RACE-determination of the resulting 5' and 3' ends of viral mRNA and cRNA, i.e. the products of transcription and replication reactions for several of the extended template vRNA constructs proves an exact recognition and sequence-specific initiation at a position equivalent to regular 3' position $\bar{1}$ : all of the various template extensions are lost in every product RNA molecule, most likely after only one round of replication.

A.2. Bicistronic (tandem) vRNA molecules carrying an additional 3' specific promoter sequence in a central position between its two genes can be used for an indirect selection method for recombinant influenza viruses:

The method described is applicable for any foreign gene (e.g. CAT) without a selection potential of its own, if inserted into the distal mRNA position (proximal vRNA position, in anti-sense orientation) behind a carrier gene (e.g. GFP) in the proximal mRNA position, able to serve as a transient selection marker. The carrier gene which is used for selection will get lost spontaneously during further propagation. These constructs are equivalent to a 3' extension of the template vRNA by a full-size gene of 750 nucleotides up to a second 3' promoter sequence, in terminal location.

While in the set of experiments shown in Fig. 2 the external 3' promoter sequence is maintained throughout as the same promoter-up variant (1104), the internal 3' promoter sequence has been varied to include a full-level promoter-up variant (pHL2270, containing promoter mutant 1104), a medium-level promoter variant (pHL2350, promoter mutant 1948), a wildtype promoter construct (pHL2629), and a construct carrying an unrelated central sequence in an otherwise identical design (pHL2300).

Due to the presence of two 3' promoter sequences in conjunction with a single 5' promoter sequence an alternating interaction between them will constitute one or the other active promoter structure (see Fig. 3). While the external location with an adjacent RNA 3' end may have a structural advantage, this appears to be compensated by the shorter distance in an interaction between the 5' sequence and the central 3' sequence in constituting the internal promoter, such that the competition between the two primarily reflects the various internal 3' promoter allele sequences used, compare Fig. 2B and activity ratios indicated above and below the lanes. Translation of the mRNA-distal gene (CAT) is only observed following an internal initiation at the bicistronic vRNA template, resulting in a spontaneous deletion of the mRNA-proximal (vRNA-distal) gene, GFP, compare right half of Fig. 3. In complementary analyses GFP fluorescence is observed initially for all of the bicistronic constructs, but gets lost on a faster rate from pHL2270 transfected and influenza infected cells (not shown), and will stay unchanged in pHL2300-treated cells. The indirect selection system based on bicistronic (tandem) molecules as designed here and demonstrated for reporter genes GFP and CAT can be used for any other gene without distinct phenotype upon insertion behind an unrelated carrier gene with properties useful in selection. - In employing that technique an initial phase of (repeated) positive selection for infected cells expressing that proximal trait (e.g. by FACS or by magneto-beads) will be followed by a second phase with negative selection, i.e. against that fraction of infected cells still exposing the same property.

A.3. Isolation of an influenza strain designed to carry 2x2 flanking ribozyme target sequences at the 5' and 3' end of vRNA segment 4 coding for hemagglutinin:

The above scheme for an indirect selection of any foreign recombinant gene behind a proximal carrier gene is further modified by deleting the carrier gene altogether. Instead, both 3' terminal promoter sequences (mutant and wildtype) follow each other at a short distance, separated only by a specific, repetitive ribozyme target sequence, - different from other target sequences to be described further below. The cDNA insert following after the second 3' promoter sequence consists mainly of a regular hemagglutinin (H7) coding sequence, however both the 5' and 3' vRNA terminal regions of the insert carry that other ribozyme target sequence (different from the first target sequence mentioned above) inserted in either location in tandem duplication (pHL2969, see Fig. 14).

Due to a superior replication supported by the promoter-up variant located in 3' external position the recombinant HA segment attached to that promoter sequence will become enriched during the first steps of viral propagation, while the originally dominating HA segment of the helper virus which is under control of a wildtype promoter sequence is consecutively reduced and finally is no longer detectable among viral progeny. This result is documented by RT-PCR analysis of consecutive viral populations as obtained in that stepwise propagation procedure, see Fig. 4.

In the next step the viral lysate is twice passaged via infection of cell culture cells (293T) that before have been DNA-transfected by plasmid pAM403 (Fig. 15). This construct has been specifically designed to express a hammerhead ribozyme with flanking sequences complementary to the repetitive GUC-containing target sequence, as present twice in between the external and internal 3' promoter signals in the recombinant HA vRNA segment, see Fig. 5. In this way the extra promoter sequence is cut off from the finally resulting recombinant HA vRNA. Its promoter-up activity was useful in achieving an initial increase in the concentration of recombinant HA vRNA over wildtype HA vRNA, and in finally excluding the latter from further propagation. However, for the same reason that high activity of the promoter variant will cause instability in the resulting viral progeny, and an effective 'substitution' at this time through ribozyme cleavage by the internally located promoter signal, wild-type or slightly improved, will restore stability to the progeny viruses, with all of their eight vRNA segments now brought back in balance to each other. Due to the ribozyme cleavage site at 26 nucleotides 3' of the wild-type promoter sequence (see Fig. 5), in the initial stage that promoter signal is situated in a vRNA-internal location, extended by a 3' terminal sequence of 26 nucleotides. According to the data presented in Fig. 1 this should cause a transient slight reduction in activity, resulting however in one step in regular viral mRNAs and cRNAs, with any initially remaining extra sequence being lost from the finally resulting recombinant HA vRNA.

Progeny viruses still carrying an external promoter-up sequence (before ribozyme treatment or due to incomplete reaction) will not cause any plaque, due to over-replication of one vRNA segment relative to all others which results in a high load of defective particles. However, progeny viruses which have lost that external promoter element due to ribozyme cleavage will yield regular plaques due to a balanced mode of replication for all eight wild-type or recombinant vRNA segments. Hence plaque purification is used for isolating a pure influenza viral strain carrying 2x2 ribozyme targets in its recombinant HA vRNA segment, with its termini reduced to the wild-type promoter sequence. The nature of the viral strain isolated has been confirmed in this regard by RT-PCR analysis, see Fig. 4.

The above isolation procedure resulting in influenza viral strains carrying 2x2 flanking ribozyme target sequences has been performed twice for the HA coding segment (segment 4) to obtain two different isolates with regard to the orientation of the ribozyme target sequences. In one of the isolates (vHM41, see SEQ. ID NO: 3) the tandem target sites have been inserted into the HA vRNA non-translated sequence both in 5' and 3' location, while according to the second design that 5' tandem target sequence has been included in an inverted orientation, such that it is now present in the cRNA 3' sequence instead (vHM42).

In another experiment the same procedure was used to isolate an influenza strain carrying 2x2 tandem target sites within the 5' and 3' flanking positions of segment 8 vRNA, i.e. coding for genes NS1 and NS2 (vHM81; see SEQ. ID NO:4). And in principle the same could be done for any other influenza segment, in particular since only the reading frame cDNA sequence has been exchanged from HA to NS, with all of the flanking elements directly responsible for that procedure remaining in place, unchanged.

A.4. Ribozyme cleavage and vRNA segment exchange using ribozyme-sensitive influenza strains as helper viruses:

In an initial model experiment a range of ribozyme type and target site locations was probed in designing a series of CAT reporter gene vRNA constructs (analysed in the presence of a surplus of wildtype helper virus) in 293T cells. While all of the ribozyme constructs adhered to the hammerhead model, with 10 to 12 nucleotides of complementary sequence flanking on either side of the GUC target site, these ribozyme constructs varied from monomer to dimer to trimer repetitions. Ribozyme containing mRNAs were synthesized *in vivo* via the basic vector plasmid pSV2-*neo,* i.e. using the efficient $p_{SVe}$ RNA polymerase II promoter element for expression, and the SV40 origin signal for plasmid amplification, in a cell line (293T or cos-1) with an incorporated SV40 T antigen gene. In addition the pSV2-*neo* mRNA includes the small, 63 nucleotide intron sequence of the SV40 early mRNA which is supposed to be spliced very slowly, thereby extending the pre-mRNA half-live in the nucleus. Each of the pSV2-*neo*-ribozyme plasmid constructs was transfected into 293T cells. Thereafter, recombinant viruses containing dimer target sites either near one end of the molecule only, or near both ends have been used for infection of the transfected cells. Relative activities of ribozyme constructs versus vRNA target sites have been measured via inactivation of CAT acetyl transfer rates in the cell lysates obtained at 8 h post infection (Fig. 6). The highest activities were observed for dimer ribozymes acting on vRNA molecules carrying 2x2 target sequences on both ends of the molecule, either in vRNA 5' and 3' location, or in vRNA 3' and cRNA 3' location, i.e. with an inversion of the target site sequence at the vRNA 5' end. Consequently, the two constructs carrying tandem ribozyme double targets within both of their non-translated vRNA flanking sequences have been used in the design of ribozyme-sensitive influenza virus strains as described above, with both variants isolated for segment 4 (HA), and one of them for segment 8 (NS). In complementary correspondence the hammerhead ribozyme plasmid used has also been constructed as a double-headed structure with flanking sequences as shown in Fig. 7 (pAM424; for its detailed structure, see Fig. 16).

The three target site-containing influenza strains isolated as described above have been analysed for their sensitivity against ribozyme cleavage by infection of 293T cells, which had been DNA-transfected 18 h earlier by ribozyme-producing plasmid pAM424, at DNA-transfection rates between 60 and 70 % (as observed in parallel transfections using GFP-expressing plasmid pAM505). Inactivation rates in these one-step control experiments were between 90% and 99% for all three ribozyme-sensitive strains, in their extent mainly depending upon the actual DNA-transfection rates achieved in individual experiments.

In the next step both isolates of HA-coding ribozyme-sensitive viruses, vHM41 and vHM42, have been used in marker-rescue experiments. Here, 293T cells have been first DNA transfected by HA-variant cDNA construct pHL2507 (see Fig. 17), followed after 18 h by vHM41 or vHM42 virus infections at moi 1. The resulting viral supernatant containing e.g. a mixture of ribozyme-sensitive vHM41 carrier virus and pHL2507/vHM41 recombinant virus is propagated in an intermediate step on MDCK cells, which also results in an increase in the fraction of recombinant viruses. Thereafter the resulting virus-containing supernatant is passaged through 293T (or cos-1) cells, which in advance have been transiently transfected by ribozyme-producing pAM424. As may be concluded from the above experiment (Fig. 6) and shown in Fig. 8 vHM41- or vHM42-derived ribozyme-sensitive HA vRNA segments are expected to be inactivated by pAM424-produced ribozyme down to a remaining level of about 1% to 10% (mainly present within cells that are infected, but not DNA-transfected).

Instead, the substitute HA vRNA which originated from pHL2507 plasmid DNA (ribozyme-resistant) becomes exclusively incorporated into progeny virions. For further purification and viral propagation these have been passaged a second time through 293T cells, again in advance DNA-transfected by pAM424, and after another amplification step on MDCK cells the resulting viral preparations have been used for RT-PCR analysis. The resulting viral populations in these marker rescue experiments only contain HA-vRNA molecules derived from pHL2507, which in their PCR analyses are of intermediate size relative to wildtype HA-vRNA, and vHM41- or vHM42-derived HA-vRNAs, respectively.

Consequently, a set of ribozyme-sensitive influenza strains with targets inserted individually into every vRNA molecule may be used for such one-step marker rescue experiments in general, i.e. for vRNA segment exchange reactions performed in a directional way for any of the eight influenza vRNA segments, without requirement for a selectable change in phenotype (genetic marker).

B. Expression of two gene products from ambisense bicistronic influenza vRNA

B.1. The influenza cRNA promoter is active in antisense viral mRNA transcription according to the cap-snatching mode of initiation:
While the vRNA template of influenza virus is known to be active in viral mRNA as well as cRNA synthesis, the cRNA template has been described so far only to produce vRNA molecules, as a second step in viral replication. The potential activity of the cRNA promoter in initiating also viral mRNA transcription has not been analysed or even suspected so far, since no antisense (vRNA) reading frame can be detected in any of the viral RNA segments. Also, no $U_5/U_6$ template sequence element is present in any of the viral cRNAs in an adjacent position to its 5' promoter structure as is the case for all viral vRNAs. This element is known to serve as a template sequence for mRNA terminal poly-adenylation, in repetitive interaction. However, when both elements are provided through reconstruction of an artificial influenza cRNA segment: a reading frame in opposite orientation (CAT), and a $U_6$ template element in 5' adjacent location, CAT expression can indeed be observed, see pHL2583 (see Fig. 18) in Fig. 9. Similar to the vRNA promoter the cRNA promoter activity is improved by (the same) promoter-up mutations, which essentially consist of basepair exchanges according to the 'corkscrew' model. This model apparently also holds for the cRNA promoter structure as analysed in a stepwise manner in Fig. 9. While the cRNA promoter has to be superior over the vRNA promoter in its initiation of replication, since the vRNA/cRNA product ratio was determined to be around 10:1 (Yamanaka et al., *In vivo* analysis of the promoter structure of the influenza virus RNA genome using a transfection system with an engineered RNA. Proc. Natl. Acad. Sci. USA 88, 5369-5373 (1991)), the cRNA promoter is observed to be inferior to the vRNA promoter in its initiation rate of transcription (compare pHL2583 with pHL1844 in Fig. 9), at least for all promoter variants tested so far.

A RACE analysis for determination of the 5' ends in pHL2583 cRNA promoter transcribed mRNAs proved this initiation to occur according to the cap-snatching mode, in complete equivalence to standard vRNA promoter controlled transcription initiation.

B.2. Development of ambisense influenza constructs for consecutive expression of two genes (GFP and CAT) from a single viral RNA:
For a bidirectional transcription and translation of influenza RNA segments the two reporter genes GFP and CAT have been arranged in opposite orientation to each other, and the flanking 5' and 3'promoter sequences (adhering to promoter-up variant 1104) had to be reconstructed to include a $U_6$ poly-adenylation element in either orientation in a 5' promoter adjacent position. This requirement necessarily resulted in a promoter-adjacent 5'-$U_6$/3'-$A_6$ complementary structure, both in the vRNA and cRNA terminal sequence (see Fig. 10), which had to be tested for its promoter activity, in either orientation. Therefore, the convergent pair of reporter genes GFP and CAT has been inserted in both orientations, such that CAT transcription is initiated by the vRNA promoter in one construct (pHL2960), and by the cRNA promoter in the other (pHL2989, Fig. 19), and vice versa for GFP expression from both ambisense constructs. In addition, also the CAT gene only has been inserted in either orientation between the 5' and 3' elements of that ambisense promoter, with CAT transcribed by the vRNA promoter in one case (pHL2959), and by the cRNA promoter in the other (pHL2957). The whole set of constructs allows for a direct comparison with corresponding reference constructs carrying a regular vRNA promoter (pHL1844) or cRNA promoter structure (pHL2583), i.e. carrying only the 5'-adjacent $U_6$ sequence element and no 3'-$A_6$ counterpart. The two groups of constructs also differ in insert size, since a single inserted gene roughly accounts for 750 nucleotides, the convergent set of two genes for 1500 nucleotides, with the distal half of both mRNAs in this case remaining untranslated, a situation unusual for influenza viral mRNAs.

As is demonstrated in Fig. 10B for CAT expression of the various ambisense constructs all of them are able to initiate transcription in both orientations, even if at different levels with regard to their vRNA and cRNA promoter-dependent expressions, and also with regard to the insert lenghts and convergent arrangements of the GFP/CAT versus CAT-only constructs. Analysis of the GFP expression rates (not shown) yields complementary results, i.e. again vRNA promoter-controlled GFP expression is superior over cRNA promoter expression of GFP. Therefore, individual ambisense clones either show an asymmetric high expression of GFP and low expression of CAT (pHL2960) or vice versa (pHL2989), depending on their orientation of reading frames with regard to the external vRNA and cRNA promoter. Fig. 10C also demonstrates successful propagation of recombinant viruses containing ambisense RNA molecules, which proves survival through amplification, packaging into virions, and expression of both mRNAs in infected MDCK cells (including besides CAT also GFP expression).

B.3. Construction of a superior promoter-up mutation, pHL1920, to be used for improved rates of cRNA promoter expression in ambisense constructs:
An extended analysis of promoter variants, in particular of complementary double exchanges according to the 'corkscrew' model yielded among others variant pHL1920 (Fig. 20) with CAT activity rates considerably above (125-130% of) the rates observed for standard promoter-up variant '1104' (as present in pHL1844). The '1920' promoter-up variant consists of altogether 5 nucleotide substitutions relative to the wildtype promoter sequence, both in the 5' promoter element (2), and the 3' promoter element (3). The structure of this variant and the whole set of complementary double exchanges is presented in Fig. 11, together with the respective CAT activity measurements, in vRNA promoter constructs. vRNA promoter-up variants also show similarly improved expression in (ambisense) cRNA constructs, even if at generally lower levels than in vRNA constructs. cRNA promoter-up expression is observed at levels similar or somewhat (2x-5x) above the *wild-type* vRNA promoter rate. while vRNA promoter-up constructs show CAT expression rates increased up to 20 or 25 times the wild-type vRNA promoter level. In either case expression rates also depend on the size of the insert, with promoter activity rates decreasing with increasing lengths of the influenza RNA molecules to be transcribed.

B.4. Influenza recombinant viruses containing a foreign gene (CAT) in covalent ambisense linkage with one of the viral genes (HA, NS1/NS2):
The principle solution in designing stable recombinant viruses based on the new properties observed for influenza transcription and replication signals consists in constructing viruses which contain a foreign gene in covalent linkage with one of the (indispensable) viral genes, in ambisense bicistronic organization. Preferably the viral gene is connected to the cRNA promoter, while vRNA promoter expression is used for expression of the foreign gene at rates considerably above the viral mRNA synthesis. The promoter-up variant chosen for constructing the ambisense RNA segment intends to bring its cRNA promoter expression (approximately) into balance with all other viral gene expression levels, which are controlled by wild-type vRNA promoters located at the termini of the seven ordinary influenza segments; the respective choice has to take into consideration the overall length of the ambisense segment.

Isolation of the ambisense recombinant virus employs an RNA polymerase I-transcribed ambisense cDNA construct, which will give rise *in vivo* to ambisense cRNA-type molecules, see Fig. 12. The plasmid DNA transfection mixture used in this step with 293T cells in addition may or may not contain four 'booster' plasmids which under $p_{CMV}$-control produce the four early influenza proteins from non-viral mRNAs: NP, plus PB1, PB2, and PA, i.e. the three subunits of viral polymerase (Pleschka et al., A plasmid-based reverse genetics system for influenza A virus J. Virol. 70, 4188-4192 (1996)), which will increase in a pre-amplification step the copy number of that ambisense viral cRNA segment. At 18 h post transfection the 293T cells are infected by a ribozyme-sensitive influenza strain, e.g. vHM41, which will supply (again) early and also late viral RNAs. The resulting supernatant which contains a mixture of vHM41 carrier virus and vHM41-derived ambisense recombinant virus (nine vRNA segments) is then passaged directly or via an intermediate step of amplification on MDCK cells onto 293T cells that have in advance been DNA-transfected by ribozyme-producing pAM424. Here, the ribozyme-sensitive vRNA segment of vHM41 will be cleaved at its 2x2 target sites by pAM424 specific ribozymes. In recombinant viruses the vRNA gene lost in this way is re-supplemented through its presence within the ambisense segment. The virus-containing supernatant is passaged for amplification and further purification through ribozyme treatment a second time on 293T cells which again have been pretreated by pAM424 DNA transfection.

Absence of ribozyme-sensitive vRNA, and presence only of ambisense RNA in RT-PCR analysis at this stage allows for further amplification on MDCK cells and a final virus stock preparation on embryonated chicken eggs. CAT assays can be used to analyse for the presence and monitor the activity of this model foreign gene through the various steps of isolation and propagation as well as document technical improvements that might be worked out for one or more of the processive stages.

C. Examples for application of helper-free, stable recombinant influenza viruses

C.1. Incorporation of reporter gene GFP in NS/GFP or HA/GFP ambisense segments:
Recombinant viruses of this type will allow to follow-up on influenza infection instantly and continuously in individual infected cells, which may also be counted or documented by fluorescence photography or FACS sorting. With improved temperature resistance (Siemering et al., Mutations that suppress the thermosensitivity of green fluorescent protein. Curr. Biol. 6, 1653-1663 (1993)) GFP expression becomes visible at 2 h after infection and shows bright fluorescence after 4 h p.i., it will be possible to follow-up on the spread of viral infection by GFP fluorescence. Stable fluorescence in abortively infected cells e. g. observed in *ex vivo* treatment of dendritic cells similarly supports a follow-up on their reincorporation into animals; other genes may be incorporated by ambisense vRNA into dendritic cells in the same way.

C.2. Construction of glycoprotein CSFV-E2 carrying influenza, helper-free:
The glycoprotein E2 of CSF virus has been incorporated previously into influenza both as an HA-anchor fusion protein within the viral envelope, and as an additional, unstable ninth vRNA segment into its genome (Zhou et al.; Membrane-anchored incorporation of a foreign protein in recombinant influenza virions. Virology, 246, 83-94 (1998)). Stabilization is now achieved through an ambisense connection with either of the regular viral RNA segments (NS or HA) which also allowed to reach a level of 100% recombinant viruses instead of an hitherto only 20% (Fig. 13), since all carrier viruses are destroyed through ribozyme action, see Figs. 8 and 12. The helper virus containing preparation has already been used successsfully as a vaccine against CSFV infection (antibody titers of 1:40000); the increase achieved in recombinant viruses allows a further improvement in that regard. Also (cost-effective) propagation in fertilized chicken eggs has become possible due to its stable incorporation of the foreign gene as a covalent ambisense construct.

C.3. Construction of hepatitis C glycoprotein-carrying recombinant influenza viruses as a candidate vaccine:

Hepatitis C virus is a close relative of CSF virus (hog cholera virus), and in particular its set of two glycoproteins, small-size E1 and larger-size E2, is closely related in structural detail and presumably also in function to the corresponding CSFV proteins. An incorporation of HCV-E2/HA fusion proteins into influenza viral envelopes has been achieved in analogy to the CSFV-E2/HA incorporation. In addition, incorporation of an anchor-fusion glycoprotein HCV-E1/HA or both together (in NS *and* HA ambisense junctions) allows further variations in constructing an influenza-based vaccine for hepatitis C. In analogy to CSFV-E2, neutralizing antibodies are expected to be directed against particular epitopes of HCV-E2, presented in essentially native conformation at the influenza viral envelope.

C.4. Stable incorporation of selected influenza T-cell epitopes in ambisense constructions:
Influenza infection is known to result in both, antibody production against that specific viral strain or indeed its epitopes that are located mainly at the surface of HA, and in an increase of specifically primed cytotoxic T-lymphocytes, stimulated by T-cell epitopes primarily located within the essentially invariable core structure of the NP protein. While the humoral response will result in life-long immunity against *that particular* strain of influenza or its epitope structures, the T-cell response will be lost or severely reduced some time afterwards, such that its specificity against influenza in general will fall below protective levels. One way in trying to increase that level of cellular immunity is to enhance the response or recruitment of influenza-specific CTL cells by increasing the level of T-cell epitopes in the infected cell and hence its presentation on the surface by MHC-I receptors. This is achieved by combining in an ambisense construct the HA gene and a series of repeated T-cell epitope sequences as present in the influenza NP gene, in a model design specific for the BALB/C mouse MHC-I allele. Here, the promoter-up expression rate is realized (in vRNA promoter-controlled initiation) for expression of the repetitive epitope polypeptide chain. Alternatively or in addition a controlled secretion of an interleukin can be achieved from recombinant influenza-infected cells, upon ambisense incorporation of the respective gene preferrably into the NS segment. The interleukin to be chosen for this purpose (IL-12 or other) is selected to enhance the longevity of influenza-specific CTL cells or its conversion into corresponding memory cells. In this way an ambisense vaccine strain against influenza itself is achieved with expected protective capacity against influenza in general.

C.5. Exchange of influenza glycoproteins against foreign viral glycoproteins (VSV-G):
The 'marker rescue' experiment described above (section A.4.), i.e. an exchange of one HA gene (ribozyme-sensitive) for another (ribozyme-resistant) can also be used for an exchange of HA for an entirely different glycoprotein, such as the vesicular stomatitis virus G protein, as long as it is attached to the HA anchor segment. Effective incorporation depends always on that C-terminal tail sequence and its interaction with underlying matrix protein M1, and therefore, all constructions consist of fusion proteins in direct analogy to CSFV-E2. VSV-G with or without a foreign anchor sequence has been shown in several other viruses to be able to substitute for the original glycoprotein and to result in infectious viruses with VSV-G specific host-ranges (e.g. in retroviruses, rabies virus, measles virus). G protein in VSV itself as well as on the surface of foreign viruses is the only glycoprotein required for all of the consecutive steps in infection. Insertion of VSV-G instead of HA in recombinant influenza viruses leaves the second glycoprotein, neuraminidase, without any function, which then will get lost spontaneously from the recombinant viruses. This will further increase the capacity for an addition of foreign genes, beyond the gain resulting from an exchange of the larger HA for the smaller VSV-G, which might be used for an addition of ambisense constructs.

The invention is further illustrated in the accompanying figures.

## Description of the figures

Fig.1. 3' nucleotide extensions of influenza vRNA template molecules:

(A) Murine B82 cells have been transfected by plasmid cDNA constructs designed to be transcribed into influenza vRNA molecules by RNA polymerase I *in vivo,* followed after 20 h by standard $FPV_{Bratislava}$ helper virus infection, at an moi of 1 to 3. In addition to reference plasmid pHL2024 (no extension), related cDNA constructs carrying extensions of 1 to 50 bp, and hence extended by 1 to 50 nucleotides at the resulting vRNA 3' ends were used in parallel transfections; template extensions are marked at the top of the figure. Cell lysates prepared at 8 h post helper virus infections were used for CAT reactions using in one round 50 pl of cell lysate each, and in further analyses 5µl and 0.5 µl of lysate (not shown). Relative yields were determined in comparison to reference plasmid pHL2024, as indicated below the figure, with calculations restricted to those CAT assays showing less than 40% of substrate consumption, in three or more independent experiments.

(B) Viral passage of B82 supernatants containing recombinant influenza virus onto MDCK cells, at an moi of 2 to 4, in average. Again at 8 h post infection cell lysates have been prepared and used for CAT assays. Relative yields as indicated below the figure have been determined in comparison to pHL2024 the same way as in (A), using 0.5 µl of cell lysate in each case. (The 50 µl CAT assays as shown here and also in the following figures intend to give an immediate impression of relative activities at always the same level, while the actual measurement data as indicated below the lanes are obtained at various appropriate enzyme concentrations relative to reference pHL2024.)

Fig. 2. Propagation of recombinant influenza viruses with tandem bicistronic vRNA:

(A) General design of expression plasmids for transient bicistronic vRNAs coding for GFP in the mRNA-proximal, and for CAT in the mRNA-distal position. Among the functional elements indicated are the human RNA polymerase I promoter ($p_{Ih}$) and murine rDNA terminator ($t_I$) sequences, both hatched, and the 5' and 3' vRNA promoter cDNA sequences, open and closed boxes, respectively. For the vRNA-internal 3' promoter signal three variant sequences have been inserted as indicated below (positions $\overline{1}$ to $\overline{15}$ refer to 3'-terminal nucleotides in the resulting monocistronic vRNAs).

(B) CAT assays as determined relative to pHL1844 (monocistronic CAT construct) after DNA transfection of 293T cells plus helper virus infection followed by one round of progeny viral propagation on MDCK cells are indicated *below* the lanes. Relative activities of the internal promoter sequences as indicated *above* the figure refer to measurements in a monocistronic *external* location of the same promoter variants (Flick *et al.*, Promoter elements in the influenza vRNA terminal structure, RNA 2, 1046-1057 (1996)). Control clone pHL2300 contains an unrelated, non-functional sequence in the central location in an otherwise identical plasmid construct.

Fig. 3. Tandem bicistronic vRNA sup-porting an alternative mode of tran-scription and replication initiation:

An additional internal 3' promoter sequence has been inserted in between both cistrons, in a vRNA-central position. Left half: bicistronic replication and transcription leading to (proximal) GFP expression. Right half: internal initiation resulting in monocistronic replication and transcription leading to (distal) CAT expression, and causing deletion of the GFP sequence from progeny molecules.

Fig. 4. Outgrowth of promoter-up recombinant vRNA versus wildtype vRNA segments in stepwise propagation of influenza virus:

RNA polymerase I transcription of transfected pHL2969 DNA results in influenza vRNA carrying (an external) promoter-up mutant '1104', and containing 2x2 ribozyme targets in flanking positions relative to its HA coding sequence. Another HA vRNA segment in wild-type configuration and originating from infecting FPV helper virus is also present in the recombinant virus preparation, initially (lane 1; 293T lysate) in surplus amounts, but reduced and finally lost entirely in consecutive steps of propagation (lanes 2 to 4; MDCK cell lysates), and in isolated strains after pAM403 ribozyme treatment for removal of the external '1104' promoter sequence (lane 5). Determination throughout by RT-PCR analyses using a pair of primers extending across the 5' inserted target site sequence, with 435 bp representing the recombinant HA segment, and 306 bp the wild-type sequence without an inserted target site sequence.

Fig.5. pAM403 ribozyme cleavage of pHL2969 derived vRNA molecules at specific target sites inserted between an external and an internal 3' promoter sequence:

The external promoter-up ('1104') signal is used for vRNA amplification within recombinant viruses and reduction of helper virus HA vRNA (Fig. 4), while the 'switch' to an internal wild-type signal guarantees stable replication of recombinant viruses. pAM403 hammerhead ribozyme RNAs are indicated in complementary binding to their target site sequences (12 and 10 nucleotides flanking the GU'C cleavage point) by straight lines flanking a central secondary structure symbol. vRNA-internal 2x2 ribozyme targets are marked by xx (see Fig. 7).

Fig. 6. Comparative cleavage analysis of model CAT vRNAs with tandem target sites in various flanking positions, by target-specific ribozymes:

293T tissue culture cells have been transiently DNA-transfected either by a single-headed hammerhead ribozyme (**s**), or a double-headed (**d**), or triple-headed (**t**) ribozyme cDNA construct, all specifically designed to hybridize to a tandem dimer target site sequence inserted in flanking positions into the CAT vRNA. All ribozyme RNAs have been expressed from the same pSV2-*neo* plasmid vector, including a pSV2-*neo* control construct without an inserted ribozyme cDNA sequence (**c**). At 20 h after DNA transfection (which reached 65% yield as measured by $p_{CMV}$-GFP transfection in parallel of the same cell culture) the 293T cells were infected by CAT recombinant viruses carrying tandem double target sequences either only in vRNA-3' position, or in both vRNA-3' and 5' positions, or in both vRNA-3' and cRNA-3' positions. Most effective among the s, d, or t-ribozymes were double-headed constructs, acting on 2x2 targets inserted in either of the two localizations described (lanes 6 and 10).

Fig. 7. Alignment of pAM424 double-headed ribozyme with one of their repetitive target sequences located within the 5' and 3' vRNA flanking regions:

The superior activity of ribozymes oriented against targets located in the 3' end of vRNA molecules over those present in the 5' end instead (not shown) is in agreement with the model for influenza vRNA transcription and replication (Lamb and Krug, Orthomyxoviridae: The viruses and their replication. In 'Virology' (B.N.Fields, D.M. Knipe, P.M.Howley, R.M.Chanock, J.L.Melnick, T.P.Monath, B.Roizman, and S.E.Straus, Eds.), 3rd ed., Vol. 1, pp. 1353-1395. Lippincott-Raven, Philadelphia (1996)), according to which influenza polymerase stays attached to the 5' end of the vRNA molecule throughout the entire or even several rounds of transcription, whereas the very 3' end repeatedly, in every initiation reaction serves as the template sequence, and consequently is no longer covered by polymerase. Superiority of a double-headed over a single-headed ribozyme has been determined earlier in this laboratory (A.Menke, Anti-Influenza Ribozyme: vRNA-Spaltung und intrazelluläre Aktivität. Dissertation Universität Giessen (1997)), but the substantial increase of vRNA inactivation rates upon incorporation of tandem target sites at both ends of the vRNA molecule instead of only one has been observed here for the first time, within that overall design.

Fig. 8: pAM424 ribozyme cleavage of resistant FPV wild-type HA vRNA and ribozyme-sensitive pHL2969-derived HA-vRNA in 293T cells infected by vHM41 after isolation from pHL2969-recombinant viral preparations. Lane 1: FPV infection of 293T cells, untreated; lane 2: FPV infection of 293T cells DNA-transfected by pAM424; lane 3: vHM41 infection of 293T cells, untreated; lane 4: vHM41 infection of 293T cells DNA-transfected by pAM424. RT-PCR analyses of purified viral progeny as in Fig. 4.

Fig. 9 Functional analysis of the influenza cRNA promoter structure:

(A) Schematic cRNA promoter ('1104') secondary structure according to the 'corkscrew' model; nucleotides involved in single or double nucleotide exchange are marked by their position.
(B) CAT analyses of 293T cell lysates after DNA transfection and FPV helper virus infection of cRNA promoter variants, in comparison to standard vRNA promoter-up mutant '1104' (pHL1844). Nucleotide substitutions divergent from the basic '1104' structure as present in pHL2583 or pHL2721 (see above) are indicated above the lanes, positions $\bar{3}$ or $\bar{8}$ as marked by a bar refer to cRNA positions counted from the 3' end. Relative CAT activities are marked below the lanes.

Fig. 10 Functional analysis of the vRNA and cRNA promoter in ambisense arrangement:

(A) Sequence organisation of the ambisense promoter cDNA construct carrying $T_6/A_6$ elements adjacent to the terminal sequence, and secondary structure predictions for the resulting cRNA and vRNA promoter signal.
(B) CAT expression data obtained from the cell lysates of 293T cell after plasmid DNA transfection and FPV infection, and (C) from cell lysates of MDCK cells after one step of viral passage. Indicated above the lanes are promoter/gene conjunctions: v = vRNA promoter; c = cRNA promoter.

Fig.11 Basepair substitutions according to the vRNA 'corkscrew' structure:

(A) 'Corkscrew' conformation of the vRNA promoter drawn against a schematic indication of interacting tripartite viral polymerase. Paired positions exchanged in individual experiments are indicated by numbers, nucleotides $\bar{3}$ or $\bar{8}$ are counted from the 3' end. pHL2024 containing promoter-up mutation '1104' is used as the reference construct (=100%) in all of the CAT assays, while pHL2428 represents the wild-type promoter structure.
(B) CAT expression data obtained after one step of viral passage in MDCK undiluted, and 50 fold diluted.

Fig. 12: Flow-chart of the isolation procedure for an ambisense recombinant influenza virus.

Fig. 13 Immuno-electron microscopy of purified influenza FPV/CSFV-E2-HA virions: Recombinant viruses exposing the foreign glycoprotein CSFV-E2 in their envelopes, which has been fused onto the HA anchor domain, are marked by anti-E2 monospecific antibody and by secondary gold-labelled (5nm) goat antibody. Recombinant viruses (16%) are present together with their FPV helper viruses.

Fig. 14: pHL2969; the exact sequence of the 4930 bps circular DNA is shown in SEQ.ID NO:1.

Fig. 15: pAM403; the exact sequence of the 5811 bps circular DNA is shown in SEQ. ID NO:2.

Fig. 16: pAM424; the exact sequence of the 5860 bps circular DNA is shown in SEQ. ID NO:5.

Fig. 17: pHL2507; the exact sequence of the 4610 bps circular DNA is shown in SEQ. ID NO:6.

Fig. 18: pHL2583; the exact sequence of the 3558 bps circular DNA is shown in SEQ. ID NO:7.

Fig. 19: pHL2989; the exact sequence of the 4343 bps circular DNA is shown in SEQ. ID NO:8.

Fig. 20: pHL1920; the exact sequence of the 3888 bps circular DNA is shown in SEQ. ID NO:9.

Hence, the present invention is based on two surprising findings, namely

1. influenza virus promoters are active when present internally in a gene;
2. the so-called cRNA, thought to be an intermediate in replication can be turned into a protein-encoding RNA by equipping it with a variant influenza virus promoter, described in the present invention.

These two observations were used to make ambisense constructs. This allows to package an additional, foreign gene into influenza virus particles. Such particles were made previously, by other methods, but proved to be unstable, and therefore useless. For use as a vaccine for example, a helper virus would have been needed as a stabilizer. Stabilization in the present invention is achieved by several means. These include the "balancing" of one of the two promoters in the ambisense biscistronic genetic construct with seven other vRNA wildtype promoters, while the additional promoter is used for high-rate expression of the foreign gene at various levels.

Thus, the present invention provides a system for expression of foreign proteins in higher eukaryotic systems. One system in particular is interesting, namely embryonated chicken eggs, as it allows cost-effective production in an automatable way (as used by most flu vaccine producers). The reason that this process is now possible, is that the foreign protein is part of a stable, engineered influenza virus particle. The virus can be designed also to rapidly monitor process improvements.

An excellent use is of course the use of the construct as a vaccine. The influenza virus particle is immunogenic and can now be equipped with foreign antigens, enabling for example the design and production of hepatitis C virus and HIV vaccines, but also of tumor vaccines. As the present invention shows, the foreign antigenic surface glycoprotein is "fused" to a C-terminal segment of influenza HA, and the antigen then is presented at the surface of influenza virus particles. In addition, these vaccines can now be made in the way standard flu vaccines are made, i.e., in embryonated chicken eggs.

**Claims**

1. A recombinant influenza virus for high-yield expression of incorporated foreign gene(s), which is genetically stable in the absence of any helper virus; wherein

   (a) at least one of the regular viral RNA segments has been exchanged for a vRNA encoding a foreign gene; and/or
   (b) at least one of the regular viral RNA segments is an ambisense RNA molecule, containing one of the standard viral genes in sense orientation and a foreign, recombinant gene in anti-sense orientation, or *vice versa,* in overall convergent arrangement.

2. The recombinant virus according to claim 1, wherein in the ambisense RNA molecule said foreign recombinant gene is covalently bound to one of the viral genes, while the original vRNA segment coding for the same gene is deleted from the recombinant virus by way of specific ribozyme cleavage.

3. The recombinant virus according to claims 1 and 2 in which one or both of the standard glycoproteins hemagglutinin and neuraminidase have been exchanged into foreign glycoprotein(s) or into fusion glycoproteins consisting of an anchor segment derived from hemagglutinin and an ectodomain obtained from the foreign source, viral or cellular, or in which such recombinant glycoprotein has been inserted as a third molecular species in addition to the remaining standard components.

4. The recombinant virus according to claims 1 to 3, in which the terminal viral RNA sequences, which are active as the promoter signal, have been varied by nucleotide substitutions in up to five positions, resulting in improved transcription rates of both the vRNA promoter as well as the cRNA promoter as present in the complementary sequence.

5. The recombinant virus according to claims 1 to 4, in which the foreign gene(s) in ambisense covalent junction with viral gene(s) code for proteins and/or glycoproteins which are secreted from cells infected with the recombinant virus.

6. The recombinant virus according to claims 1 to 4, in which the foreign gene(s) in ambisense covalent junction with viral gene(s) code for proteins or artificial polypeptides designed to support an efficient presentation of inherent epitopes at the surface of (abortively) infected cells, for stimulation of a B cell and /or T cell response.

7. A method for the production of recombinant influenza viruses as defined in claims 1 to 6 comprising

   (a) RNA polymerase I synthesis of recombinant vRNAs *in vivo,* in antisense, sense or ambisense design,
   (b) followed by infection with an influenza carrier strain constructed to include flanking ribozyme target sequences in at least one of its viral RNA segments, and
   (c) thereafter selective vRNA inactivation through ribozyme cleavage.

8. A method of constructing influenza carrier strains carrying one or more ribozyme target sites (of type one) in vRNA flanking positions comprising

   (a) RNA polymerase I synthesis of recombinant vRNAs *in vivo,* carrying two different 3' promoter sequences in tandem, which are separated by a second type of ribozyme target sequence, and which carry the said internal ribozyme target sites of type one;
   (b) followed by infection of an influenza wildtype strain;
   (c) thereafter amplification through simple steps of viral propagation; and
   (d) finally isolation through removal of their external 3' promoter sequence by ribozyme cleavage through infection of cells expressing ribozyme type 2, followed by plaque purification.

9. A ribozyme-sensitive influenza carrier strain obtainable by the method of claim 8.

10. A pharmaceutical composition comprising a recombinant virus according to claims 1 to 6.

11. Use of a recombinant virus according to claims 1 to 6 for preparing a medicament for vaccination purposes.

**12.** The use according to claim 11, wherein the medicament

(a) is suitable against influenza and/or against other infections;
(b) is present in form of inactivated preparations; and/or
(c) is present in form of live recombinant (attenuated) viruses.

**13.** Use of a recombinant virus according to claims 1 to 6 as vector systems in somatic gene therapy, for transfer and expression of foreign genes into cells (abortively) infected by such viruses, either in *ex vivo* or *in vivo* application schemes.

**14.** A method for the production of proteins or glycoproteins which comprises utilizing a recombinant influenza virus according to claims 1 to 6 as expression vector.

**15.** The method of claim 14, wherein the production is performed in cell culture cells or in fertilized chicken eggs.

FIG. 1

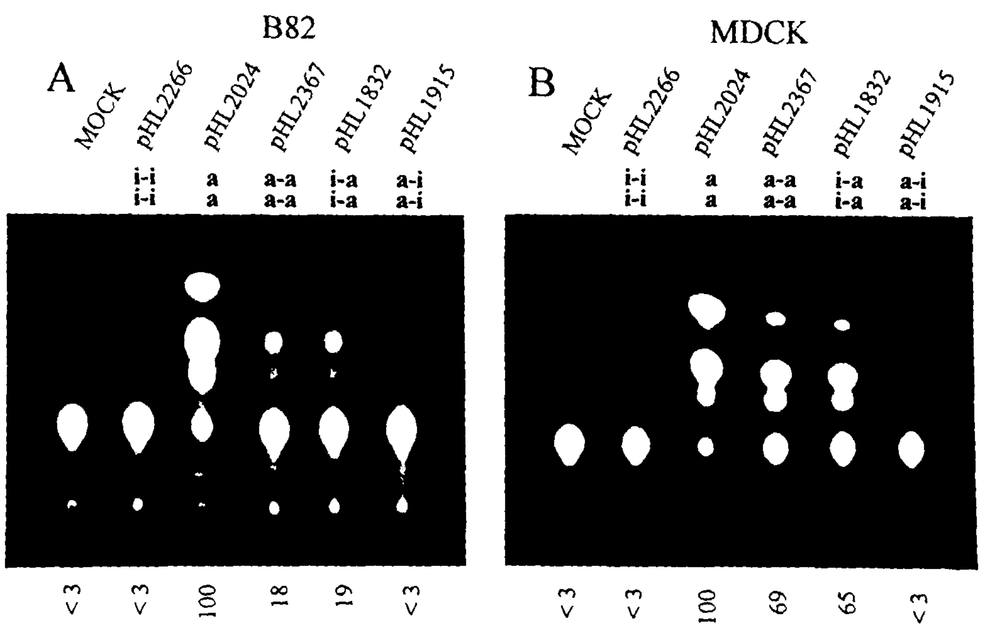
B82
MDCK
A
B
MOCK
pHL2266
pHL2024
pHL2367
pHL1832
pHL1915
i-i i-i
a a
a-a a-a
i-a i-a
a-i a-i
<3
<3
100
18
19
<3
<3
<3
100
69
65
<3

FIG. 2

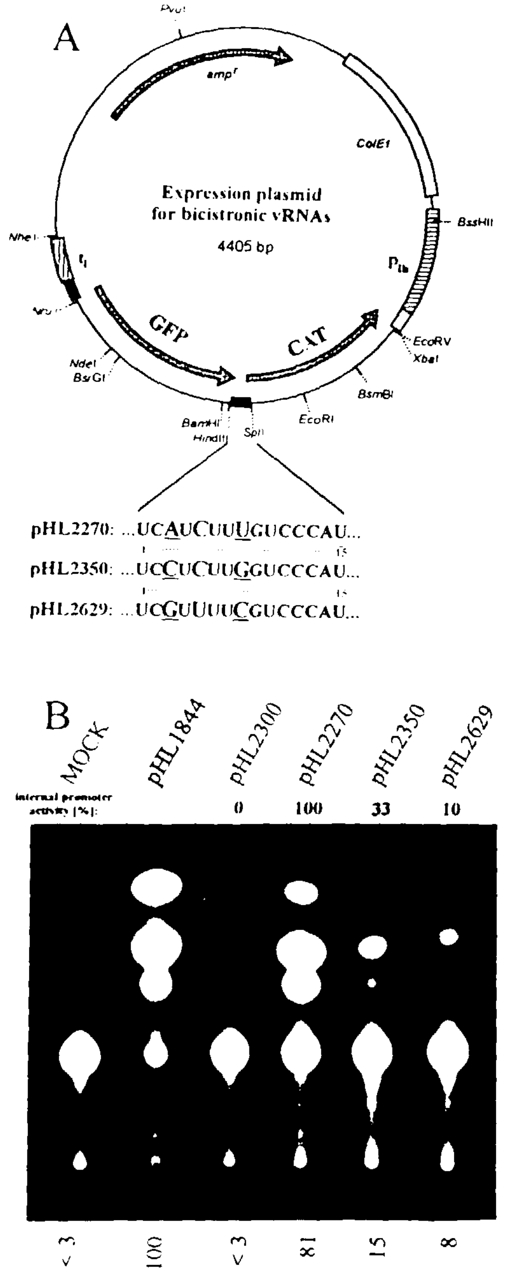
A
ampr
ColE1
Expression plasmid
for bicistronic vRNAs
4405 bp
BssHII
Nhe I
GFP
CAT
EcoRV
XbaI
NdeI
BsrGI
BsmBI
EcoRI
BamHI
HindIII
SpII
pHL2270: ...UCAUCUUUGUCCCAU...
pHL2350: ...UCCUCUUGGUCCCAU...
pHL2629: ...UCGUUUUCGUCCCAU...
B
MOCK
pHL1844
pHL2300
pHL2270
pHL2350
pHL2629
internal promoter activity [%]:
0
100
33
10
< 3
100
< 3
81
15
8

FIG. 3

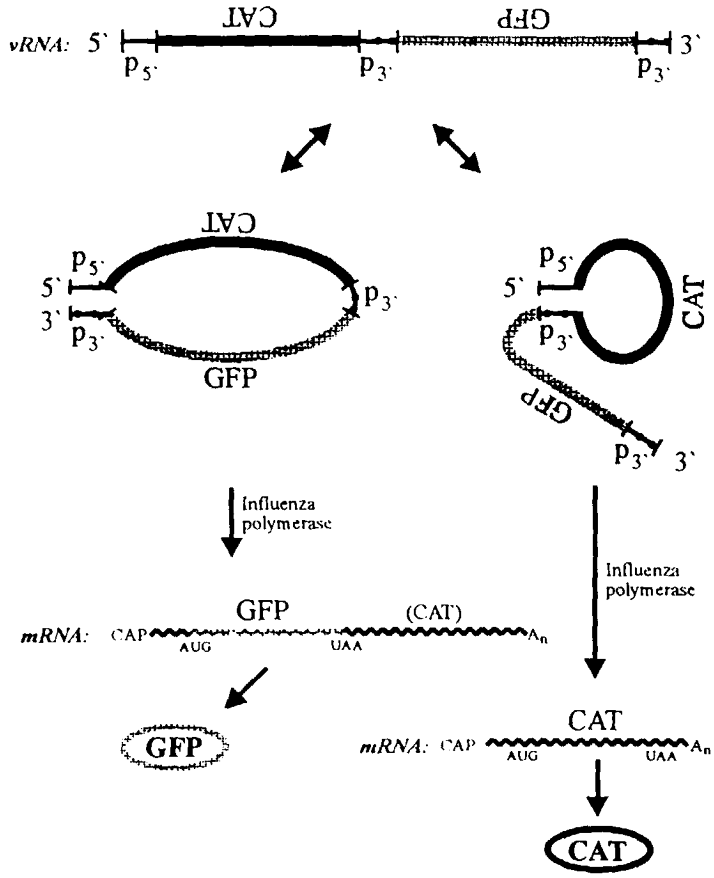
CAT
GFP
vRNA: 5'
3'
$P_{5'}$
$P_{3'}$
$P_{3'}$
CAT
$P_{5'}$
5'
3'
$P_{3'}$
$P_{3'}$
GFP
$P_{5'}$
5'
$P_{3'}$
CAT
GFP
$P_{3'}$
3'
Influenza polymerase
Influenza polymerase
GFP
(CAT)
mRNA: CAP
AUG
UAA
$A_n$
GFP
CAT
mRNA: CAP
AUG
UAA
$A_n$
CAT

FIG. 4

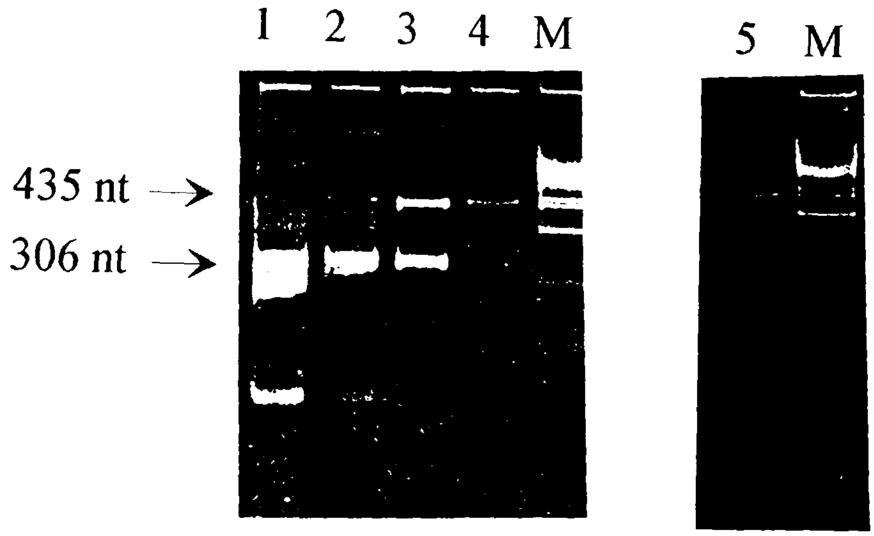
1 2 3 4 M
5 M
435 nt
306 nt

FIG. 5

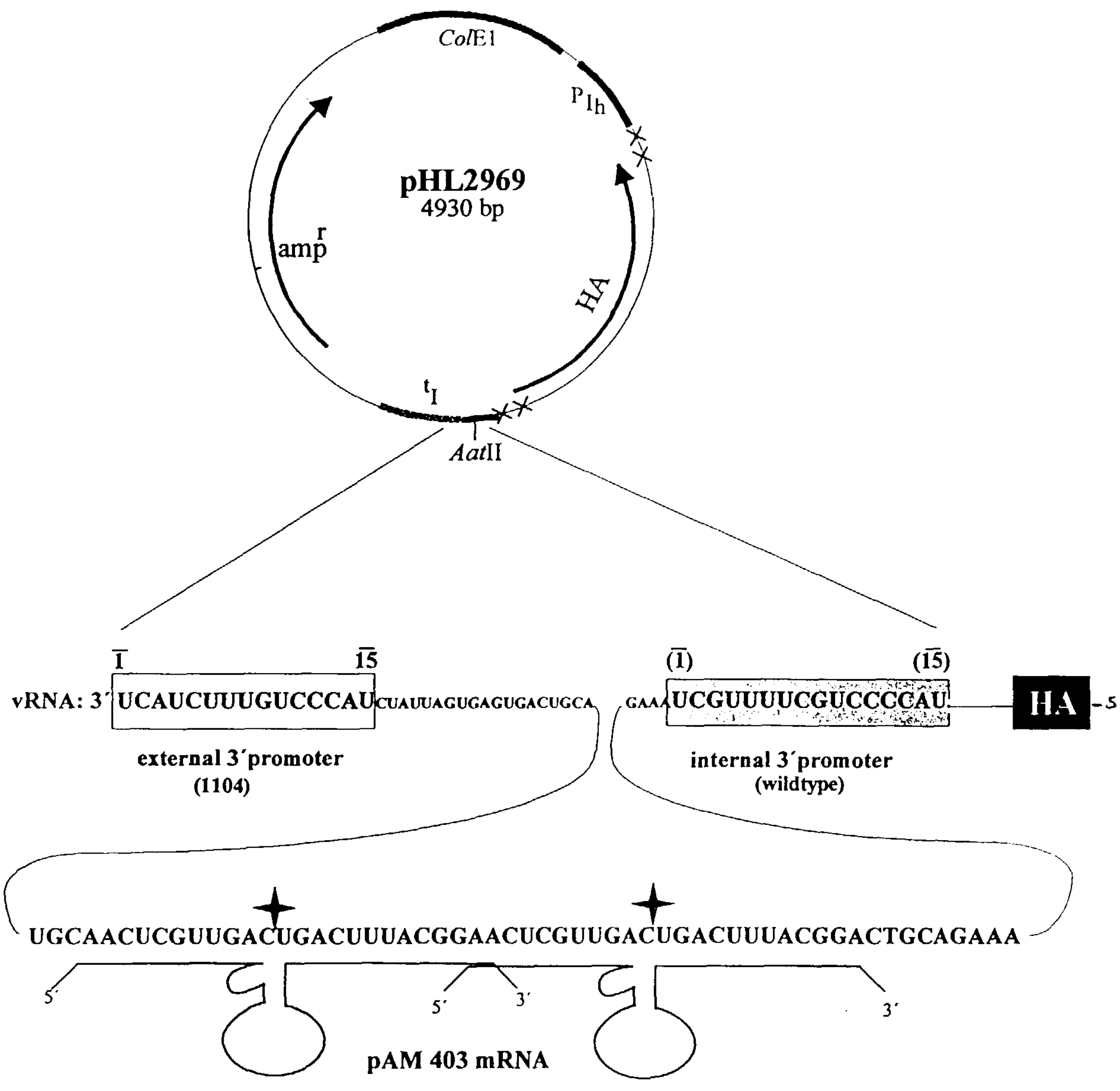

ColE1
$P_{Ih}$
pHL2969
4930 bp
amp^r
HA
$t_I$
AatII
1
15
(1)
(15)
vRNA: 3´ UCAUCUUUGUCCCAU CUAUUAGUGAGUGACUGCA
GAA UCGUUUUCGUCCCCAU
HA -5
external 3´promoter
(1104)
internal 3´promoter
(wildtype)
UGCAACUCGUUGACUGACUUUACGGAACUCGUUGACUGACUUUACGGACTGCAGAAA
5´
5´
3´
3´
pAM 403 mRNA

FIG. 6

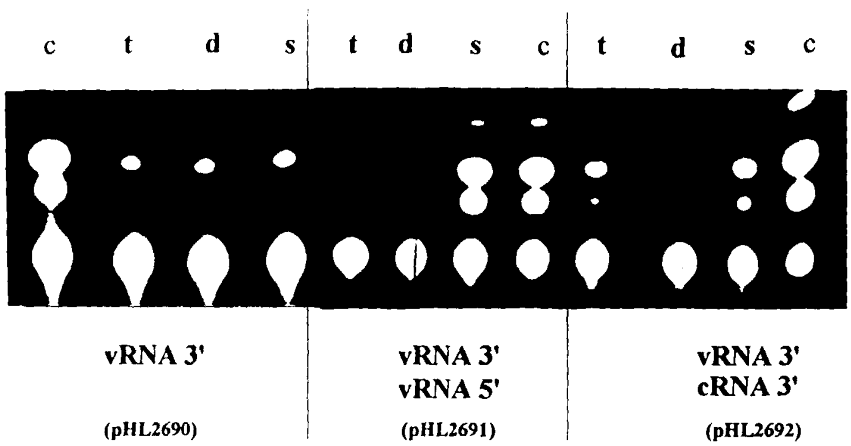
c t d s t d s c t d s c
vRNA 3'
(pHL2690)
vRNA 3'
vRNA 5'
(pHL2691)
vRNA 3'
cRNA 3'
(pHL2692)

FIG. 7

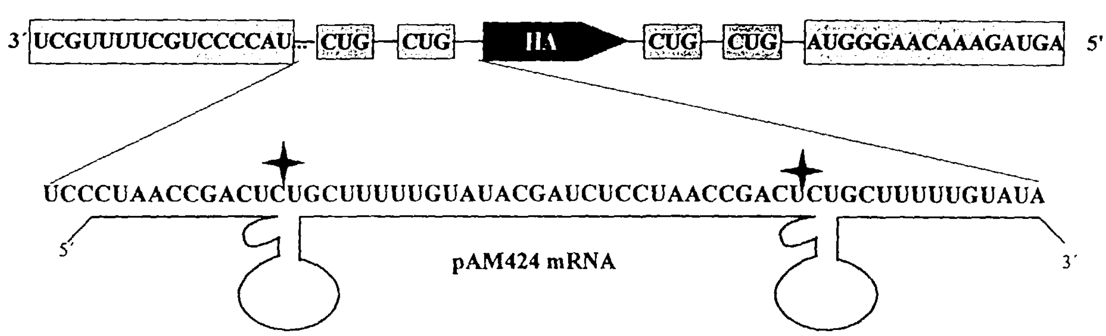
3' UCGUUUUCGUCCCCAU CUG CUG HA CUG CUG AUGGGAACAAAGAUGA 5'
UCCCUAACCGACUCUGCUUUUUGUAUACGAUCUCCUAACCGACUCUGCUUUUUGUAUA
5'
pAM424 mRNA
3'

FIG. 8

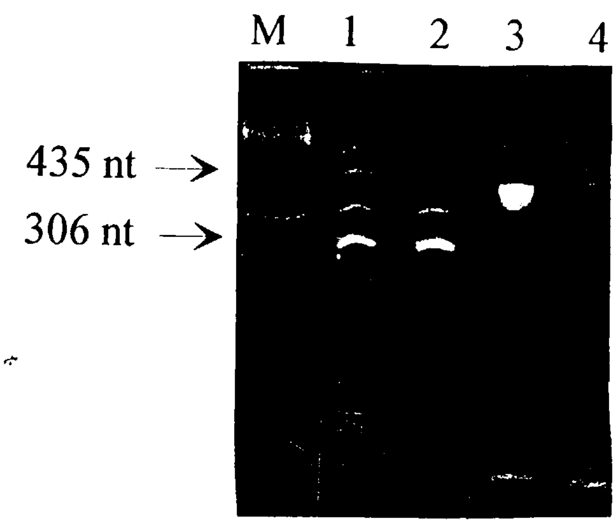

FIG. 9

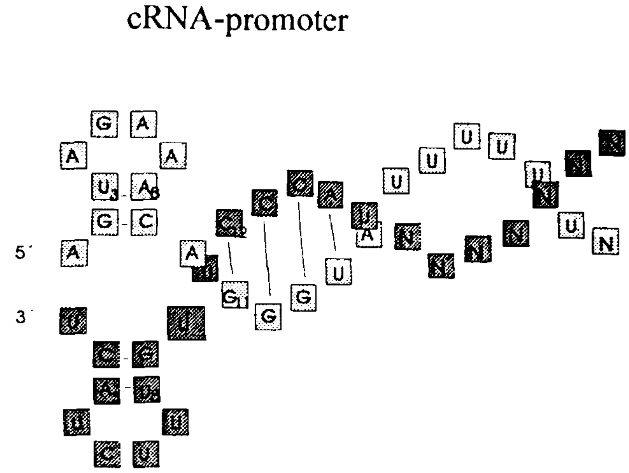
cRNA-promoter
5´
3´

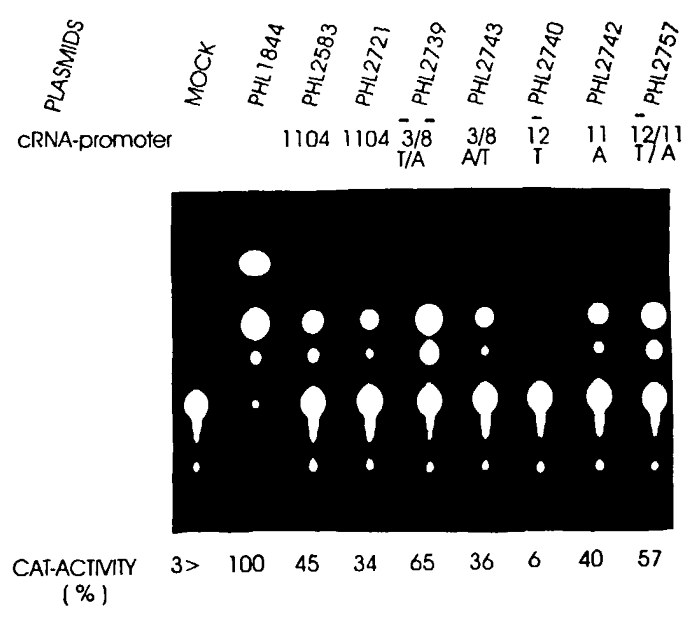
PLASMIDS
MOCK
PHL1844
PHL2583
PHL2721
PHL2739
PHL2743
PHL2740
PHL2742
PHL2757
cRNA-promoter
1104 1104 3/8 T/A 3/8 A/T 12 T 11 A 12/11 T/A
CAT-ACTIVITY (%)
3> 100 45 34 65 36 6 40 57

FIG. 10

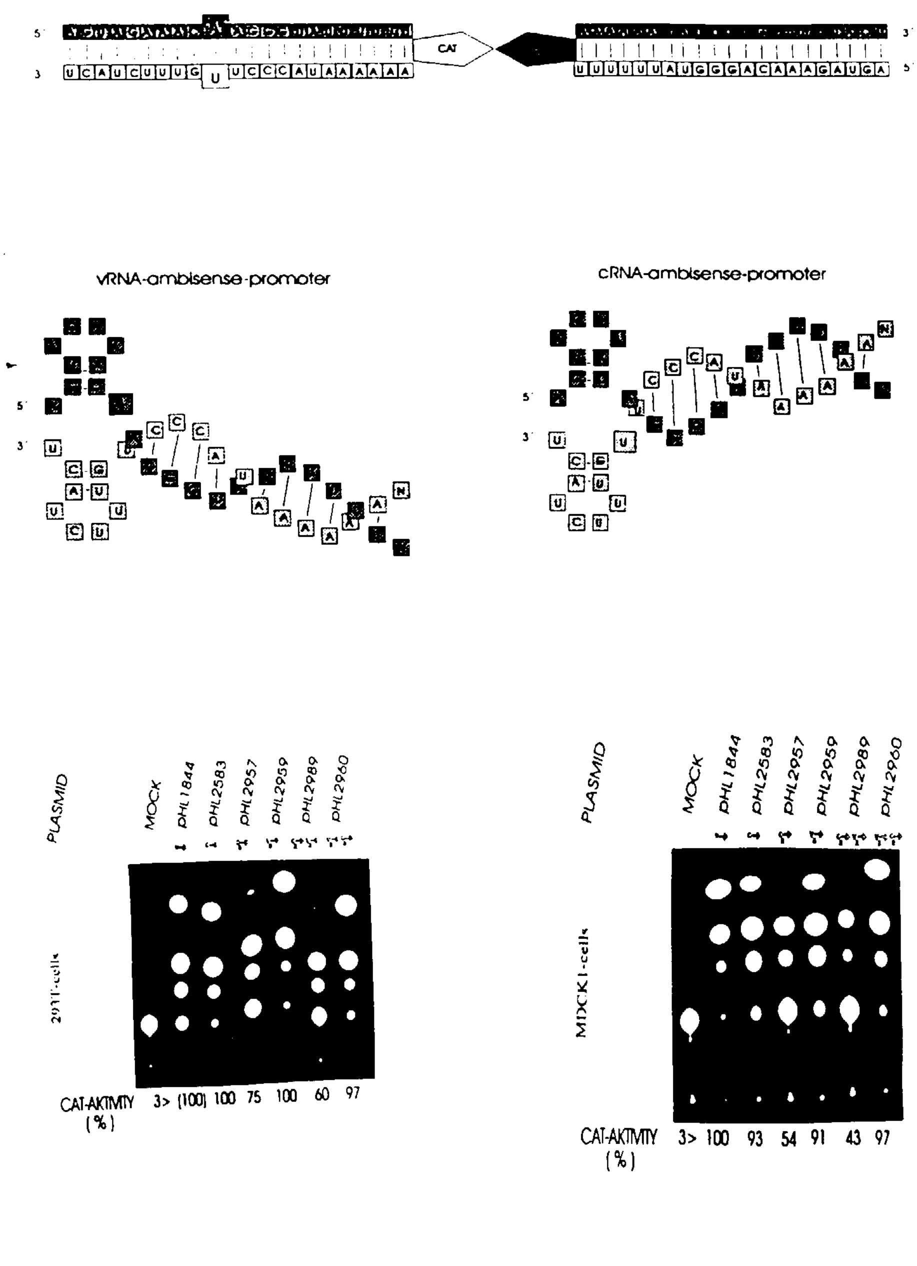
CAT
vRNA-ambisense-promoter
cRNA-ambisense-promoter
PLASMID
MOCK
DHL1844
DHL2583
DHL2957
DHL2959
DHL2989
DHL2960
CAT-AKTIVITY (%)
3> (100) 100 75 100 60 97
CAT-AKTIVITY (%)
3> 100 93 54 91 43 97

# FIG. 11

A

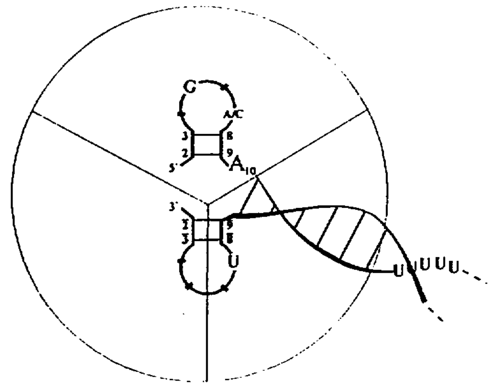


| bp-variant / position | G - C | A - U | C - G | U - A |
|---|---|---|---|---|
| 2 - 9 | pHL2024<br>100% | pHL1921<br>41% | pHL2003<br>< 3% | pHL2004<br>< 3% |
| 3 - 8 | pHL2002<br>30% | pHL1920<br>121% | pHL1148<br>39% | pHL2024<br>100% |
| $\bar{2}$ - $\bar{9}$ | pHL1945<br>11% | pHL1946<br>30% | pHL2024<br>100% | pHL1923<br>28% |
| $\bar{3}$ - $\bar{8}$ | pHL2428<br>6% | pHL2024<br>100% | pHL1948<br>33% | pHL1922<br>97% |

B

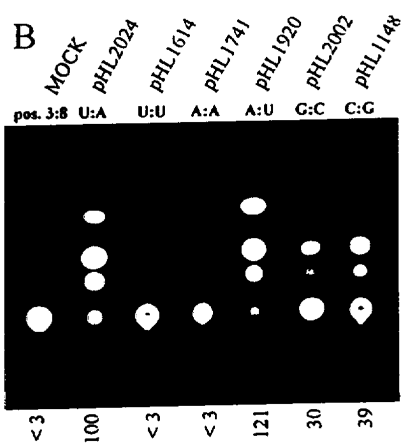


C

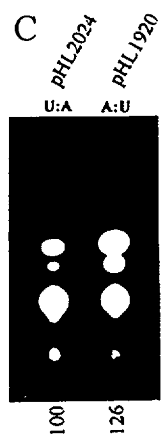

FIG. 12

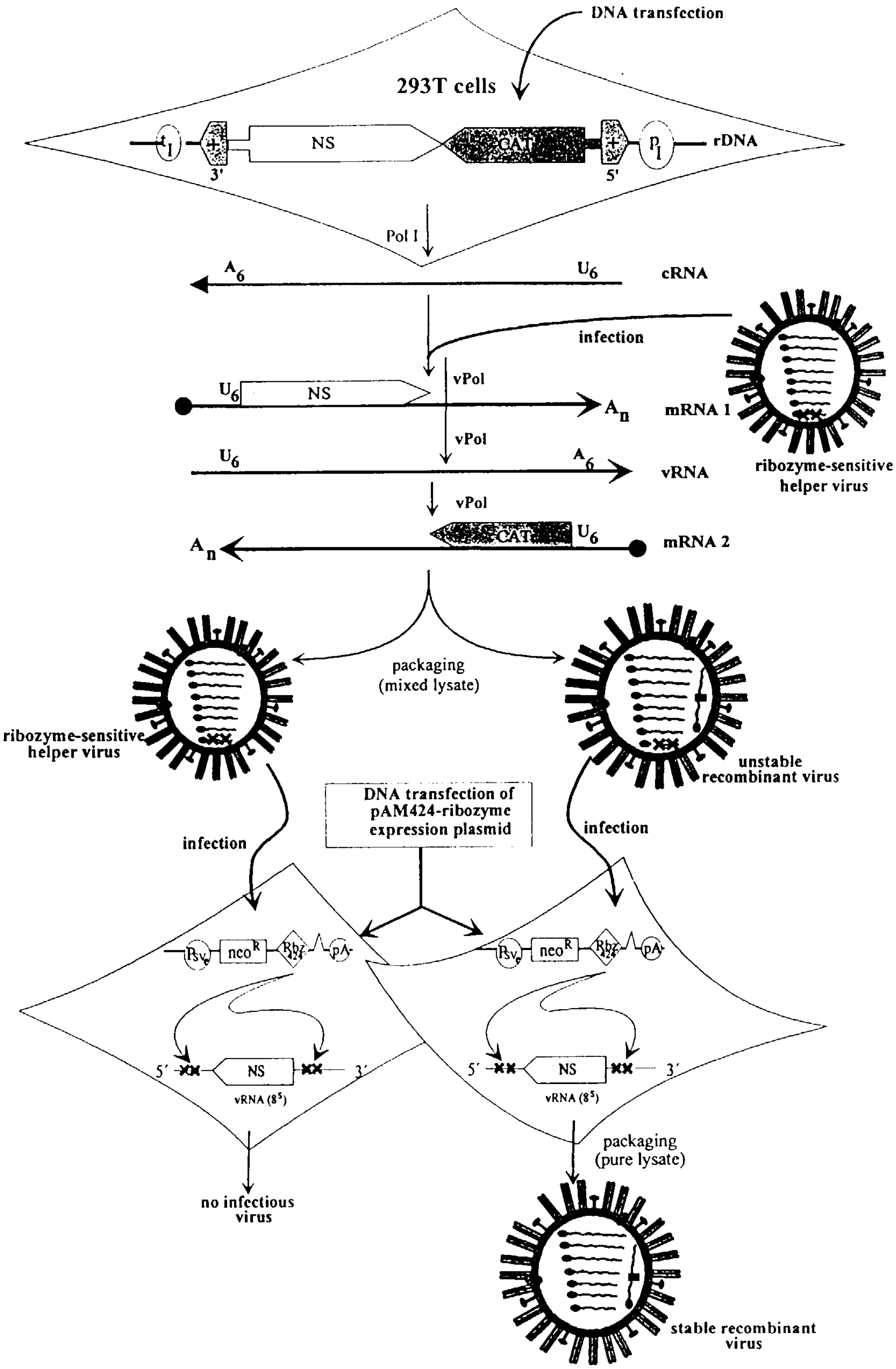
DNA transfection
293T cells
NS
CAT
3'
5'
rDNA
Pol I
cRNA
infection
vPol
NS
mRNA 1
vPol
vRNA
ribozyme-sensitive
helper virus
vPol
CAT
mRNA 2
packaging
(mixed lysate)
ribozyme-sensitive
helper virus
unstable
recombinant virus
DNA transfection of
pAM424-ribozyme
expression plasmid
infection
infection
NS
vRNA (8S)
NS
vRNA (8S)
packaging
(pure lysate)
no infectious
virus
stable recombinant
virus

FIG. 13

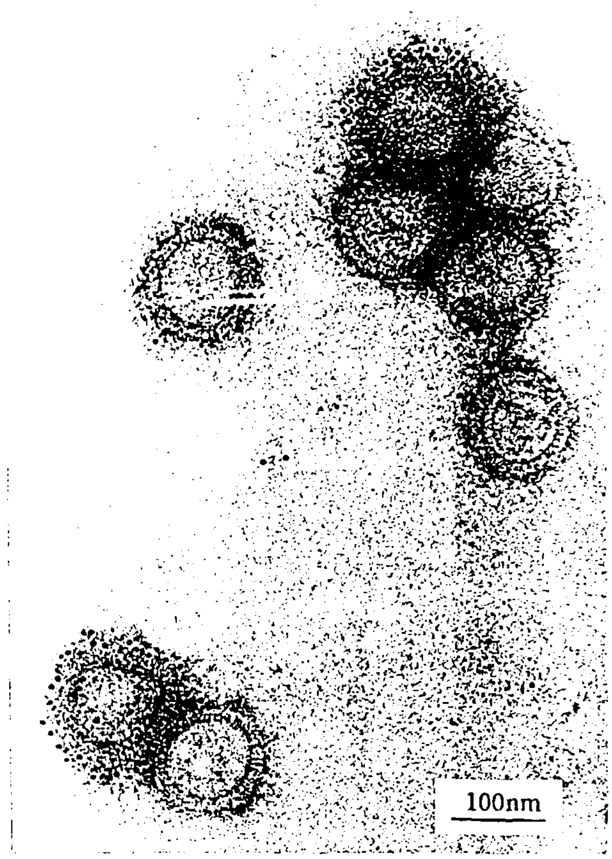

FIG. 14

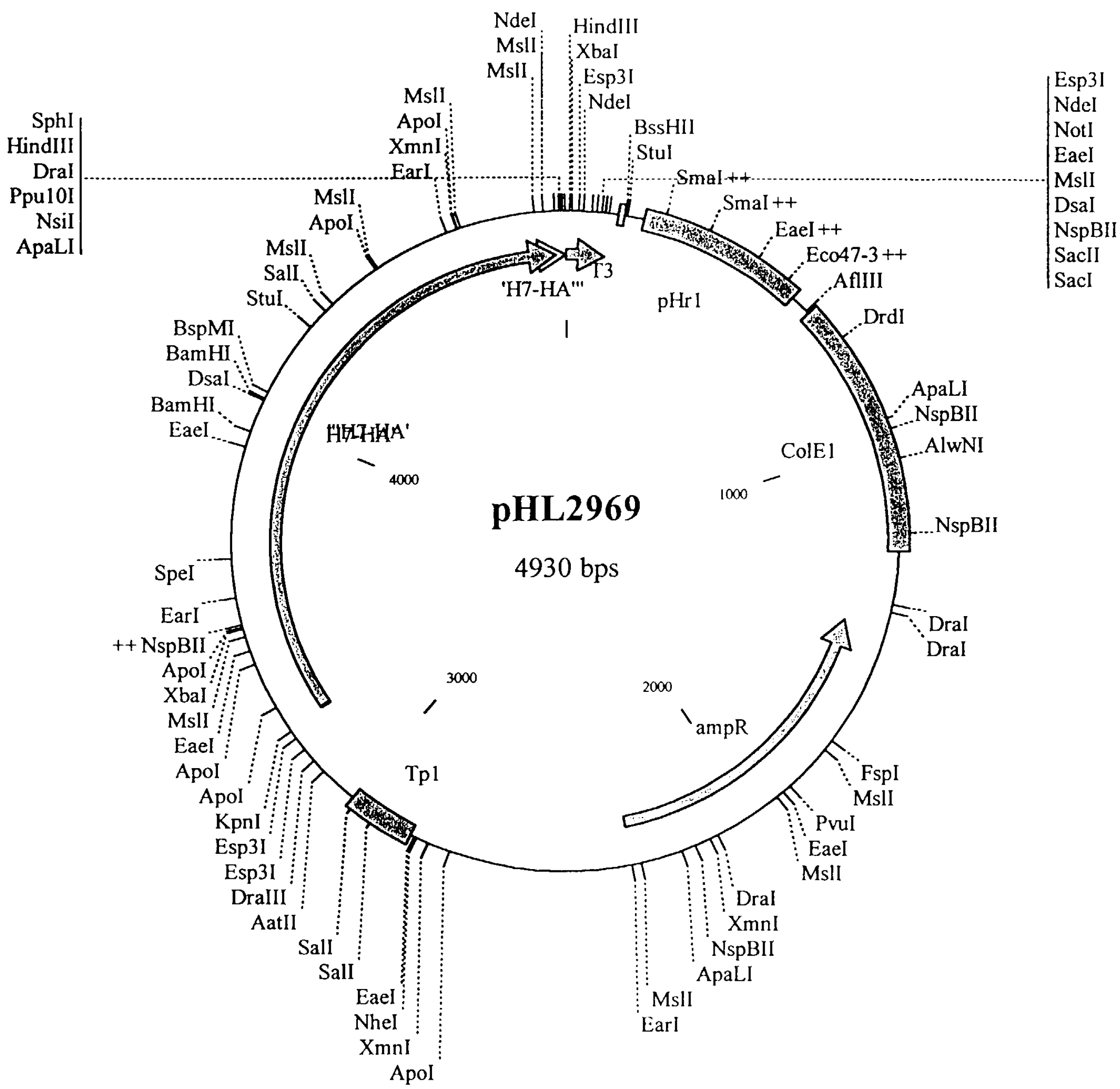
pHL2969
4930 bps
'H7-HA'''
T3
pHr1
ColE1
'H7-HA'
ampR
Tp1
1000
2000
3000
4000
SphI
HindIII
DraI
Ppu10I
NsiI
ApaLI
Esp3I
NdeI
NotI
EaeI
MslI
DsaI
NspBII
SacII
SacI
NdeI
MslI
MslI
HindIII
XbaI
Esp3I
NdeI
BssHII
StuI
SmaI ++
SmaI ++
EaeI ++
Eco47-3 ++
AflIII
DrdI
ApaLI
NspBII
AlwNI
NspBII
DraI
DraI
FspI
MslI
PvuI
EaeI
MslI
DraI
XmnI
NspBII
ApaLI
MslI
EarI
EaeI
NheI
XmnI
ApoI
SalI
SalI
AatII
DraIII
Esp3I
Esp3I
KpnI
ApoI
ApoI
EaeI
MslI
XbaI
ApoI
++ NspBII
EarI
SpeI
EaeI
BamHI
DsaI
BamHI
BspMI
StuI
SalI
MslI
ApoI
MslI
MslI
ApoI
XmnI
EarI

FIG. 15

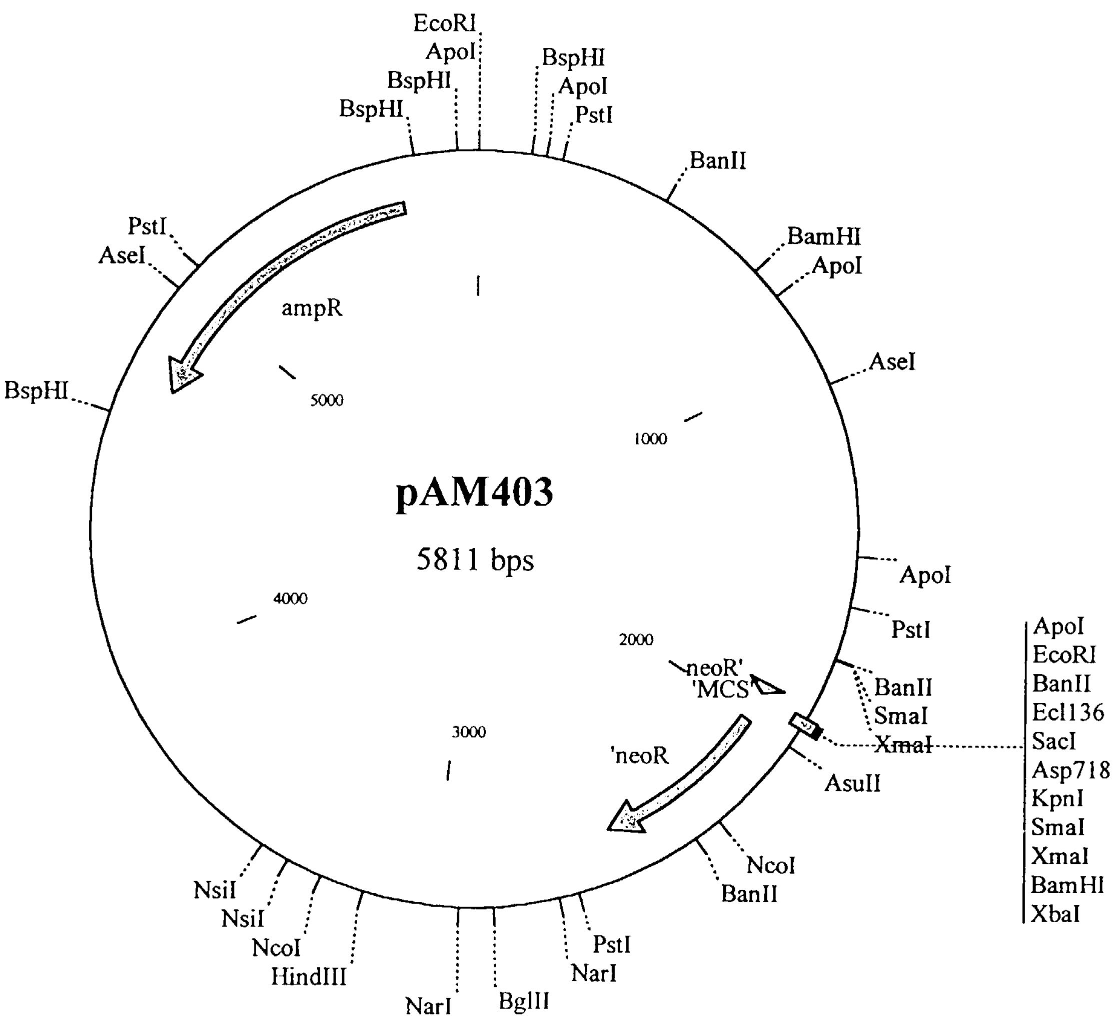
EcoRI
ApoI
BspHI
BspHI
BspHI
ApoI
PstI
BanII
BamHI
ApoI
AseI
PstI
AseI
BspHI
ampR
5000
1000
pAM403
5811 bps
4000
2000
ApoI
PstI
neoR'
'MCS'
BanII
SmaI
XmaI
3000
'neoR
AsuII
NcoI
BanII
NsiI
NsiI
NcoI
HindIII
NarI
BglII
NarI
PstI
ApoI
EcoRI
BanII
Ecl136
SacI
Asp718
KpnI
SmaI
XmaI
BamHI
XbaI

FIG. 16

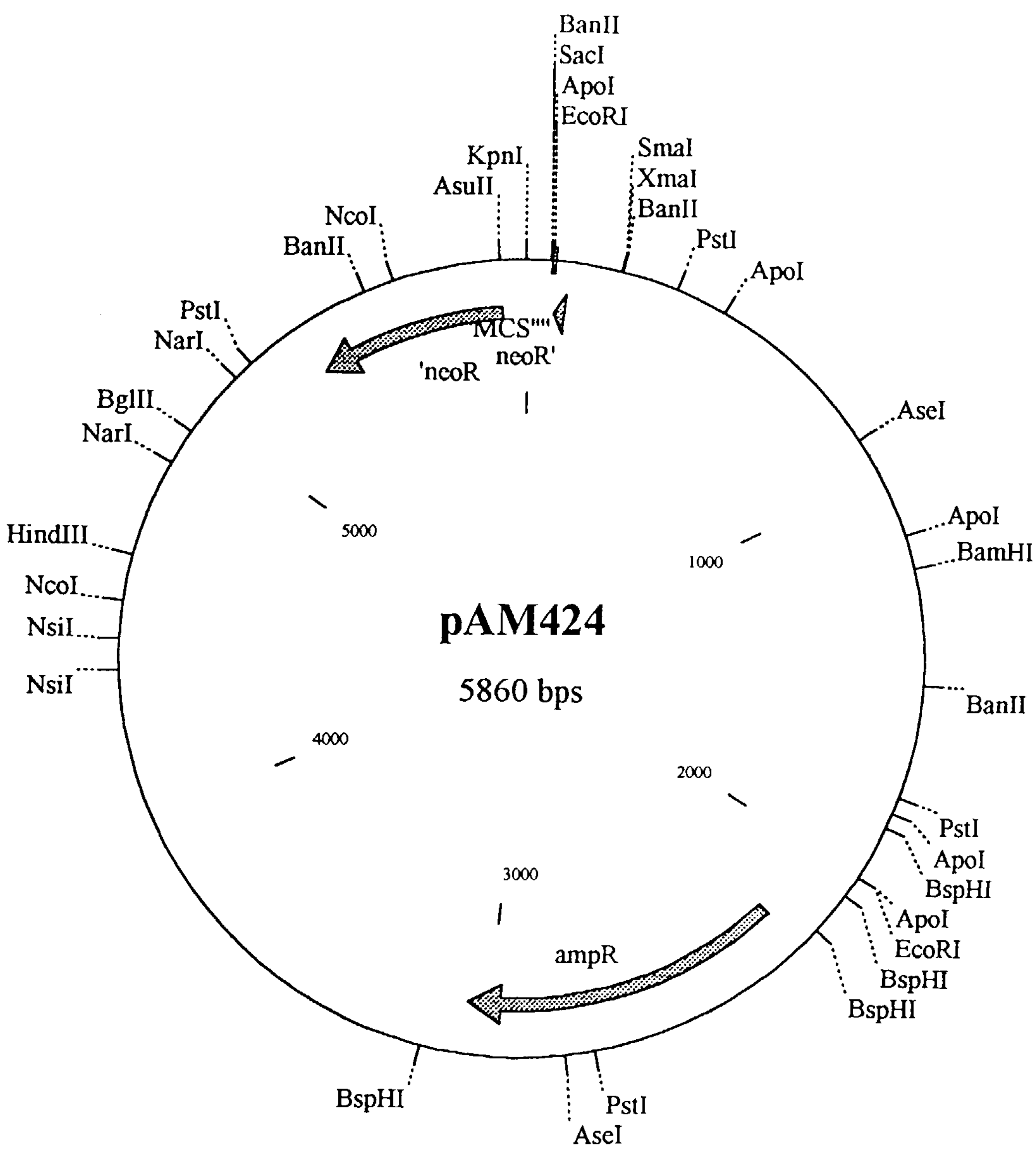
BanII
SacI
ApoI
EcoRI
KpnI
AsuII
SmaI
XmaI
BanII
NcoI
BanII
PstI
ApoI
PstI
NarI
MCS
neoR'
'neoR
BglII
NarI
AseI
ApoI
BamHI
HindIII
5000
1000
NcoI
NsiI
NsiI
pAM424
5860 bps
BanII
4000
2000
PstI
ApoI
BspHI
3000
ApoI
EcoRI
ampR
BspHI
BspHI
BspHI
PstI
AseI

FIG. 17

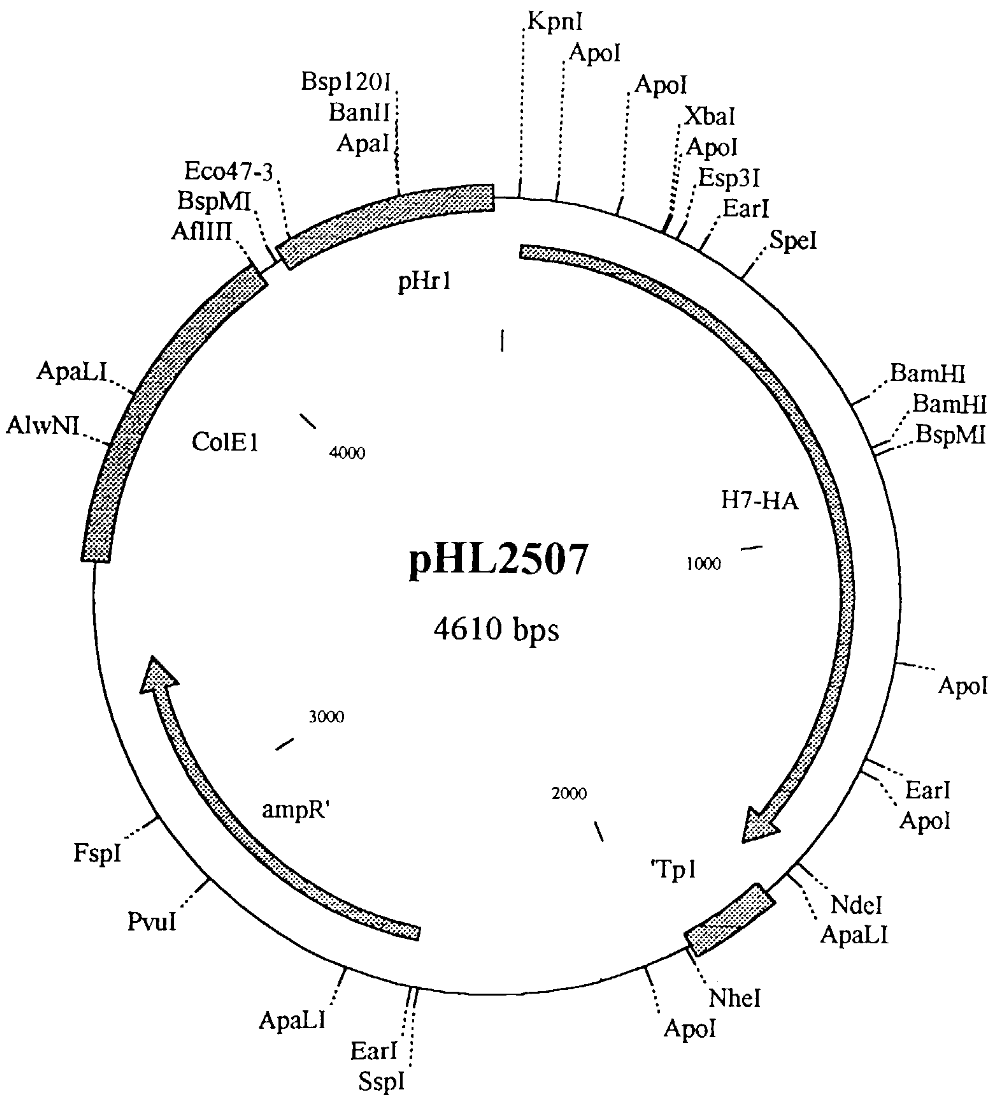
KpnI
ApoI
ApoI
XbaI
ApoI
Esp3I
EarI
SpeI
BamHI
BamHI
BspMI
ApoI
EarI
ApoI
NdeI
ApaLI
NheI
ApoI
EarI
SspI
ApaLI
PvuI
FspI
AlwNI
ApaLI
AflIII
BspMI
Eco47-3
ApaI
BanII
Bsp120I
pHr1
ColE1
4000
H7-HA
1000
pHL2507
4610 bps
3000
2000
ampR'
'Tp1

FIG. 18

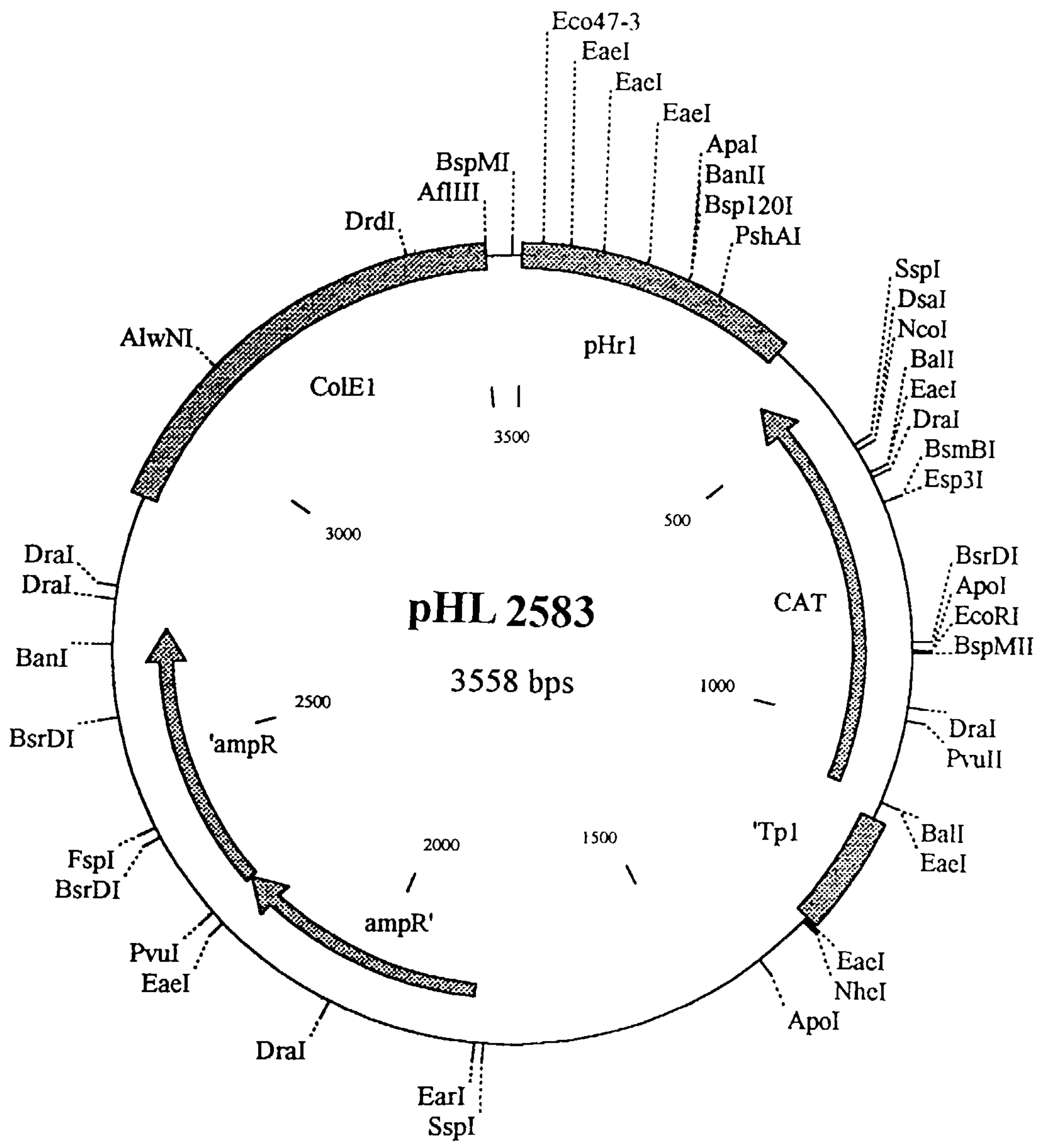
Eco47-3
EaeI
EacI
EaeI
ApaI
BanII
Bsp120I
PshAI
BspMI
AflIII
DrdI
AlwNI
SspI
DsaI
NcoI
BalI
EaeI
DraI
BsmBI
Esp3I
BsrDI
ApoI
EcoRI
BspMII
DraI
PvuII
BalI
EacI
EacI
NheI
ApoI
EarI
SspI
DraI
PvuI
EaeI
FspI
BsrDI
BsrDI
BanI
DraI
DraI
pHr1
ColE1
CAT
'Tp1
ampR'
'ampR
3500
500
3000
1000
2500
2000
1500
pHL 2583
3558 bps

FIG. 19

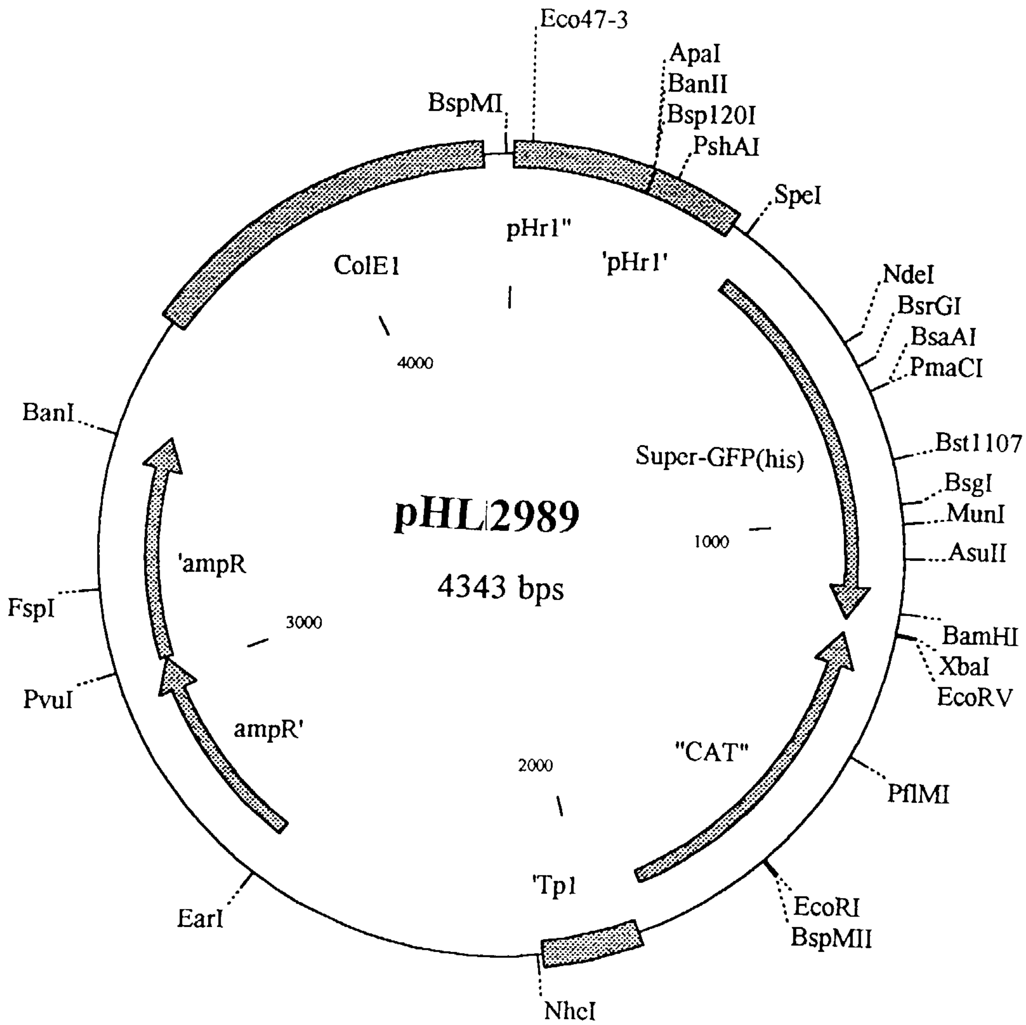
Eco47-3
ApaI
BanII
Bsp120I
PshAI
SpeI
NdeI
BsrGI
BsaAI
PmaCI
Bst1107
BsgI
MunI
AsuII
BamHI
XbaI
EcoRV
PflMI
EcoRI
BspMII
NheI
EarI
PvuI
FspI
BanI
BspMI
pHr1"
'pHr1'
ColE1
4000
Super-GFP(his)
pHL2989
4343 bps
1000
'ampR
3000
ampR'
2000
"CAT"
'Tp1

FIG. 20

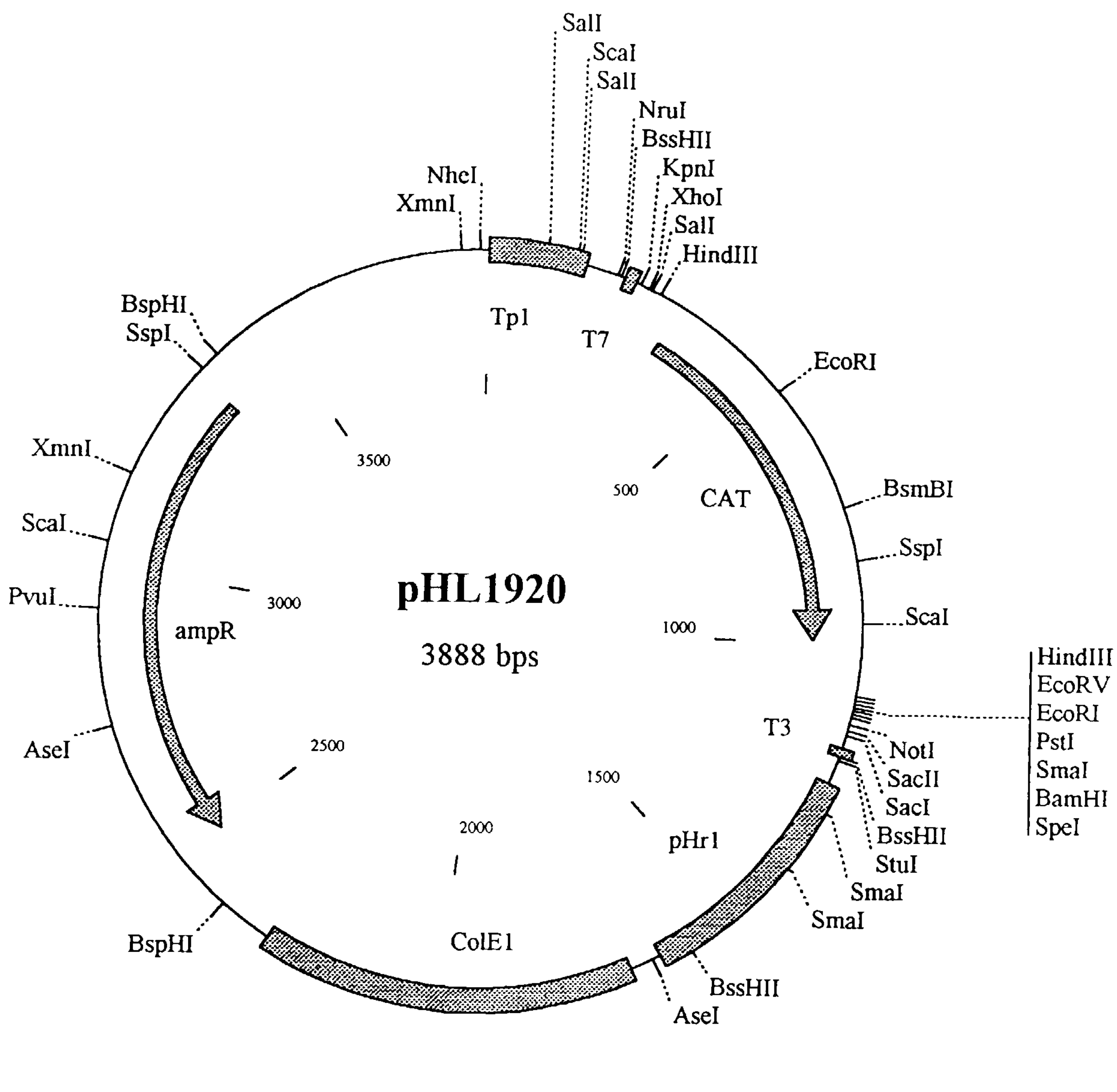
SalI
ScaI
SalI
NruI
BssHII
KpnI
XhoI
SalI
HindIII
NheI
XmnI
BspHI
SspI
Tp1
T7
EcoRI
XmnI
3500
500
CAT
BsmBI
ScaI
SspI
PvuI
3000
pHL1920
3888 bps
1000
ScaI
ampR
HindIII
EcoRV
EcoRI
PstI
SmaI
BamHI
SpeI
T3
NotI
AseI
2500
1500
SacII
SacI
BssHII
2000
pHr1
StuI
SmaI
SmaI
BspHI
ColE1
BssHII
AseI

European Patent Office

# PARTIAL EUROPEAN SEARCH REPORT

which under Rule 45 of the European Patent Convention shall be considered, for the purposes of subsequent proceedings, as the European search report

Application Number EP 99 10 4519

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| X<br><br>Y | DE 197 09 512 A (HOBOM GERD PROF DR DR)<br>10 September 1998 (1998-09-10)<br>* the whole document *<br>--- | 1,3,4,<br>7-14<br>2,5,6,15 | C12N15/86<br>C12N7/01<br>C12N5/10<br>A61K39/00<br>A61K39/145<br>A61K48/00 |
| X<br><br>Y | WO 91 03552 A (SINAI SCHOOL MEDICINE)<br>21 March 1991 (1991-03-21)<br>* figure 11; example 7 *<br>--- | 1,10-15<br><br>2,5,6 | |
| Y | TAKASE H. ET AL: "Antibody responses and protection in mice immunized orally against influenza virus."<br>VACCINE,<br>vol. 14, no. 17/18, 1996, pages 1651-1656, XP002110225<br>* page 1652, left-hand column, paragraph 1 *<br>--- | 15 | |
| X | ZHOU Y. ET AL.: "Membrane-anchored incorporation of a foreign protein in recombinant Influenza virions."<br>VIROLOGY,<br>vol. 246, 20 June 1998 (1998-06-20), pages 83-94, XP002110226<br>* the whole document *<br>---<br>-/-- | 1,10-14 | TECHNICAL FIELDS SEARCHED<br>C12N<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 26 July 1999 | Mandl, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# INCOMPLETE SEARCH SHEET C

Application Number
EP 99 10 4519

Although claim 13 and claim 14, as far as an in vivo application is concerned, are directed to a method of treatment of the human/animal body (Article 52(4) EPC), the search has been carried out and based on the alleged effects of the compound/composition.

European Patent Office

# PARTIAL EUROPEAN SEARCH REPORT

Application Number
EP 99 10 4519

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| X | PALESE P. ET AL.: "Negative-strand RNA viruses: Genetic engineering and applications." PROC. NATL. ACAD. SCI. U.S.A., vol. 93, October 1996 (1996-10), pages 11354-11358, XP000196755 * page 11354, right-hand column, last paragraph - page 11356, right-hand column, paragraph F * | 1,10-14 | |
| X | ZOBEL A. ET AL.: "RNA polymerase I catalysed transcription of insert viral cDNA." NUCLEIC ACIDS RESEARCH, vol. 21, no. 16, 1993, pages 3607-3614, XP002110227 * page 3607, right-hand column, paragraph 2 * * page 3612, right-hand column, paragraph 2 - page 3613, left-hand column, line 1 * * page 3614, left-hand column, paragraph 2 * | 1,10 | TECHNICAL FIELDS SEARCHED |

EPO FORM 1503 03.82 (P04C10)

## ANNEX TO THE EUROPEAN SEARCH REPORT ON EUROPEAN PATENT APPLICATION NO. EP 99 10 4519

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-1999

| Patent document cited in search report | Publication date | Patent family member(s) | Publication date |
|---|---|---|---|
| DE 19709512 A | 10-09-1998 | NONE | |
| WO 9103552 A | 21-03-1991 | US 5166057 A | 24-11-1992 |
| | | AT 126272 T | 15-08-1995 |
| | | AU 636916 B | 13-05-1993 |
| | | AU 6411890 A | 08-04-1991 |
| | | CA 2065245 A | 01-03-1991 |
| | | DE 69021575 D | 14-09-1995 |
| | | DE 69021575 T | 14-12-1995 |
| | | DK 490972 T | 30-10-1995 |
| | | EP 0490972 A | 24-06-1992 |
| | | ES 2075901 T | 16-10-1995 |
| | | GR 90100639 A | 30-12-1991 |
| | | JP 5500607 T | 12-02-1993 |
| | | PT 95124 A | 18-04-1991 |
| | | US 5252289 A | 12-10-1993 |
| | | US 5578473 A | 26-11-1996 |
| | | US 5854037 A | 29-12-1998 |
| | | US 5840520 A | 24-11-1998 |
| | | US 5786199 A | 28-07-1998 |
| | | US 5820871 A | 13-10-1992 |

EPO FORM P0459

For more details about this annex : see Official Journal of the European Patent Office, No. 12/82